# FASCICULE DE BREVET EUROPEEN

(11) **EP 0 783 903 B1**
(45) Date de publication et mention de la délivrance du brevet: **07.06.2000**
(21) Numéro de dépôt: 97440001.2
(22) Date de dépôt: 08.01.1997
(51) Int. Cl.: A61N 5/04

(54) **Sonde, notamment sonde urétrale, pour le chauffage de tissus par micro-ondes et pour la mesure de température par radiométrie**
Sonde, insbesondere Harnröhrensonde, zum Heizen mittels Mikrowellen von Gewebe, und zum Messen der Temperatur mittels Radiometrie
Probe, particularly urethral probe, heating tissues by means of microwaves and radiometric temperature measuring

(30) Priorité: 12.01.1996 FR 9600451
(43) Date de publication de la demande: 16.07.1997
(73) Titulaire: BRUKER SA, 67160 Wissembourg (FR)
(72) Inventeur: Brevard, Christian, 67160 Wissembourg (FR); Weiss, Michel, 67240 Gries (FR); Loewenguth, Bernard, 67160 Wissembourg (FR); Mabire, Jean-Pierre, 67500 Haguenau (FR)
(74) Mandataire: Nuss, Pierre

(56) Documents cités:
- EP-A- 0 317 067
- EP-A- 0 648 515
- WO-A-93/17756
- WO-A-95/01814

## Description

La présente invention concerne le domaine des techniques d'hyperthermie ou de thermothérapie, plus particulièrement les sondes et les antennes destinées à être introduites dans un conduit ou une cavité du corps humain, notamment de faible dimension, tel que l'urètre prostatique, et a pour objet une sonde pour le chauffage de tissus par micro-ondes et pour la mesure de température par radiométrie micro-onde, notamment au niveau de la prostate.

La thermométrie par radiométrie micro-ondes, dont le principe est bien connu de l'homme du métier, et dont un procédé de mesure est décrit dans le brevet français n° 2 650 390, peut avantageusement mettre en oeuvre un capteur micro-ondes qui est constitué par l'antenne d'émission micro-ondes utilisée en réception.

On connaît également déjà de très nombreuses sondes du type précité, notamment intra urétrale, présentant une antenne du type filaire, constituée par l'extrémité dénudée d'un câble coaxial.

Toutefois, ces sondes connues réalisent un chauffage inhomogène des tissus environnants, en concentrant exagérément une part importante de l'énergie rayonnée dans un volume restreint, et ne présentent pas un volume chauffé coïncidant avec un volume de tissus spécifiques à chauffer, tel que par exemple la prostate.

De plus, la forme du diagramme de rayonnement micro-onde de ces sondes étant différente de celle de leur diagramme de réception radiométrique, de telles sondes ne peuvent être utilisées de manière optimale en tant qu'antennes de réception pour la mesure radiométrique.

En vue de pallier les inconvénients précités, il a été proposé de réaliser et de mettre en oeuvre des sondes, notamment urétrale, pour le chauffage par émission micro-ondes et la mesure de température par radiométrie micro-onde, comprenant, d'une part, une antenne allongée formée par au moins une portion de conducteur enroulée de manière hélicoïdale sur un support diélectrique allongé présentant une extrémité avant et une extrémité arrière, d'autre part, des moyens de liaison électrique pour le transfert de signaux micro-ondes vers et à partir de ladite antenne, reliés à un générateur et à un radiomètre extérieurs correspondants, et, enfin, un cathéter recouvrant ladite antenne et, le cas échéant, au moins la partie des moyens de liaison électrique proximale de ladite antenne.

De telles sondes sont notamment décrites dans la demande de brevet français n° 2 711 066 et dans la demande de brevet européen n° 0 648 515 au nom de la demanderesse.

Or, dans le cadre de nouveaux protocoles de thermothérapie utilisant des températures nettement plus élevées, il est nécessaire d'effectuer à la fois une nécrose en profondeur des tissus prostatiques, et une nécrose de la paroi urétrale afin de créer une véritable loge prostatique. De plus, afin de détruire les terminaisons nerveuses superficielles pour éviter toute sensation de douleur, et la micro-circulation sanguine s'opposant à la montée en température par le système de thermorégulation de l'organisme, des puissances élevées (70 à 90W) sont utilisées.

Il en résulte une élévation de température très importante desdites portions de conducteur rayonnantes et de la ligne d'alimentation qui n'autorise pas, immédiatement après une séquence d'émission donnée, de réaliser des mesures radiométriques fiables et significatives par l'intermédiaire desdites portions de conducteur.

Il a donc été proposé d'adopter la solution préconisée pour les sondes à antennes filaires et de disposer lesdites portions de conducteur rayonnantes dans un manchon parcouru par un liquide de thermostatisation.

Néanmoins, ce liquide de thermostatisation entourant l'antenne constituée par lesdites portions de conducteur, forme un écran entre ladite antenne et les tissus environnants. Ledit liquide de thermostatisation absorbe une partie du bruit thermique émis par les tissus environnants et la ligne d'alimentation et capté en phase de mesure radiométrique par l'antenne. De plus, le liquide de thermostatisation émet un bruit thermique parasite s'ajoutant au bruit thermique émis par les tissus chauffés.

De plus, cet écran liquide sépare d'avantage l'antenne de mesure des tissus environnants et réduit donc le gradient de température entre ces derniers et ladite antenne, d'où résulte une baisse de la sensibilité de mesure de la température par radiométrie.

En outre, le liquide de thermostatisation circulant entre l'antenne et la prostate, présente l'inconvénient de placer les lieux où le champ électrique, donc le dépôt d'énergie, est maximum dans le film d'eau et non pas dans les tissus prostatiques. L'efficacité du traitement s'en trouve diminuée.

La présente invention a pour objet de pallier notamment l'ensemble des inconvénients précités.

A cet effet, elle a pour objet une sonde pour le chauffage de tissus par micro-ondes et pour la mesure de température par radiométrie micro-onde, telle que définie à la revendication 1.

L'invention sera mieux comprise, grâce à la description ci-après, qui se rapporte à des modes de réalisation préférés, donnés à titre d'exemple non limitatif, et expliqué avec référence aux dessins schématiques annexés, dans lesquels :
la figure 1 est une vue en élévation latérale d'une sonde selon l'invention ;
la figure 2 est une vue en élévation latérale et en coupe selon le plan de la figure 1 de l'antenne et du support faisant partie de la sonde représentée sur cette figure, selon un premier mode de réalisation de l'invention ;
la figure 3 est une vue en élévation latérale du corps support portant l'antenne représentée à la figure 2 ;
la figure 4 est une vue en élévation latérale et en coupe selon le plan de la figure 1 de l'antenne et du support faisant partie de la sonde représentée sur cette figure, selon une première variante d'un deuxième mode de réalisation de l'invention ;
la figure 5 est une vue en élévation latérale et en coupe selon un plan perpendiculaire au plan de la figure 1 de l'antenne et du support faisant partie de la sonde représentée sur cette figure, selon une première variante d'un deuxième mode de réalisation de l'invention ;
la figure 6 est une vue de l'antenne et du corps support, identique à celle de la figure 4, selon une seconde variante d'un deuxième mode de réalisation de l'invention ;
la figure 7 est une vue de l'antenne et du support identique à celle de la figure 5, selon une seconde variante d'un deuxième mode de réalisation de l'invention ;
la figure 8 est une vue en élévation latérale du corps support portant l'antenne représentée sur les figures 6 et 7 ;
les figures 9A, 9B, 9C et 9D sont des vues en coupe, respectivement selon A-A, B-B, C-C et D-D, du support diélectrique central représenté à la figure 8 ;
la figure 10 est une vue en élévation latérale, à une échelle différente et partiellement en coupe d'une sonde selon l'invention, comprenant une antenne réalisée selon un troisième mode de réalisation de l'invention ;
la figure 11 est une vue en élévation latérale et en coupe selon le plan de la figure 1 de l'antenne et du corps support faisant partie de la sonde représentée sur cette figure ;
la figure 12 est une vue à une échelle différente du détail A de la figure 11 ;
les figures 13A à 13C sont des vues en perspective montrant différentes étapes successives d'un mode de fabrication de l'antenne d'une sonde ;
la figure 14 est une vue en élévation latérale et partiellement en coupe d'un ensemble antenne(s) / corps support similaire à celui représenté à la figure 10, mais comprenant deux antennes, et,
la figure 15 est une vue similaire à celle de la figure 13A montrant la première étape de la réalisation des antennes représentées à la figure 14.

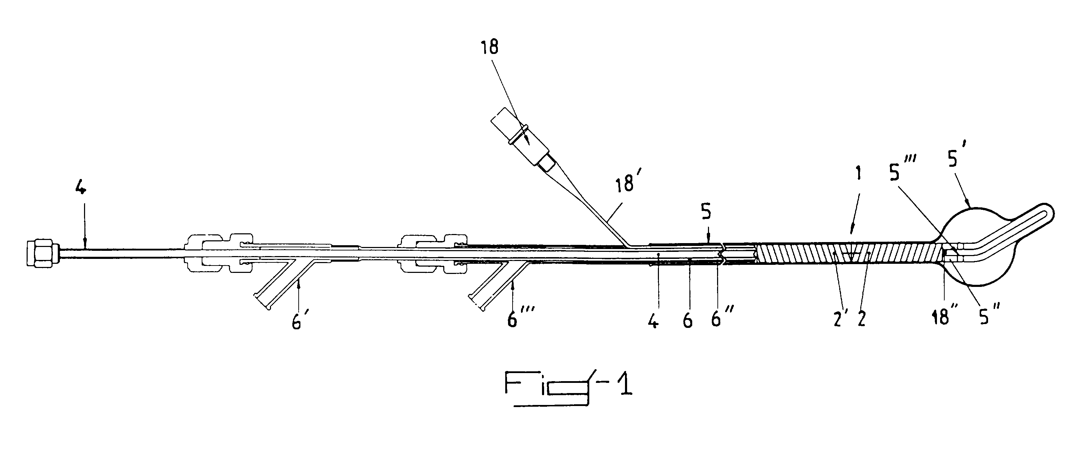

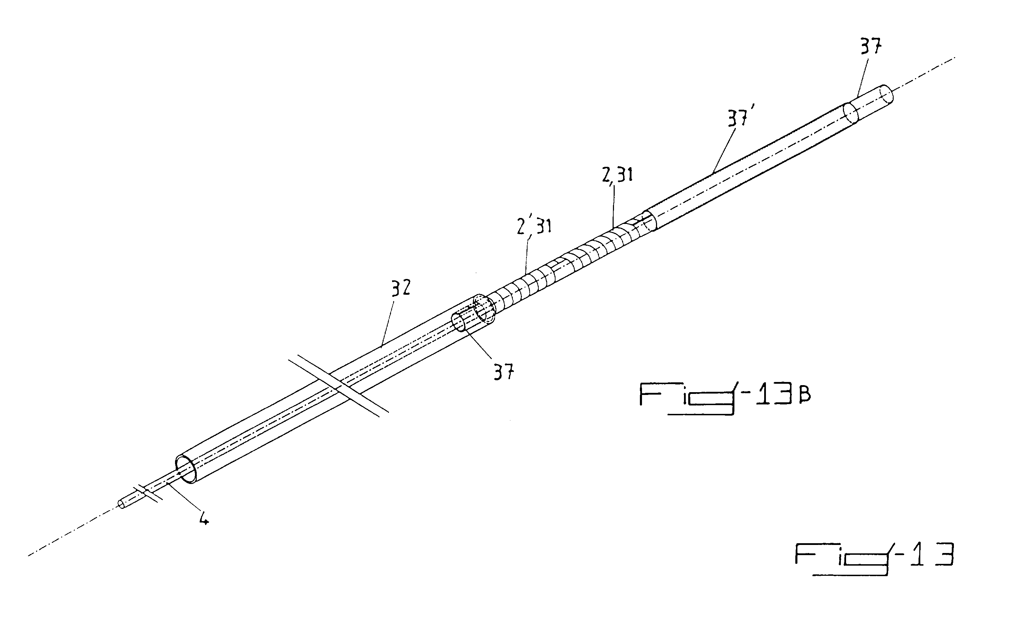

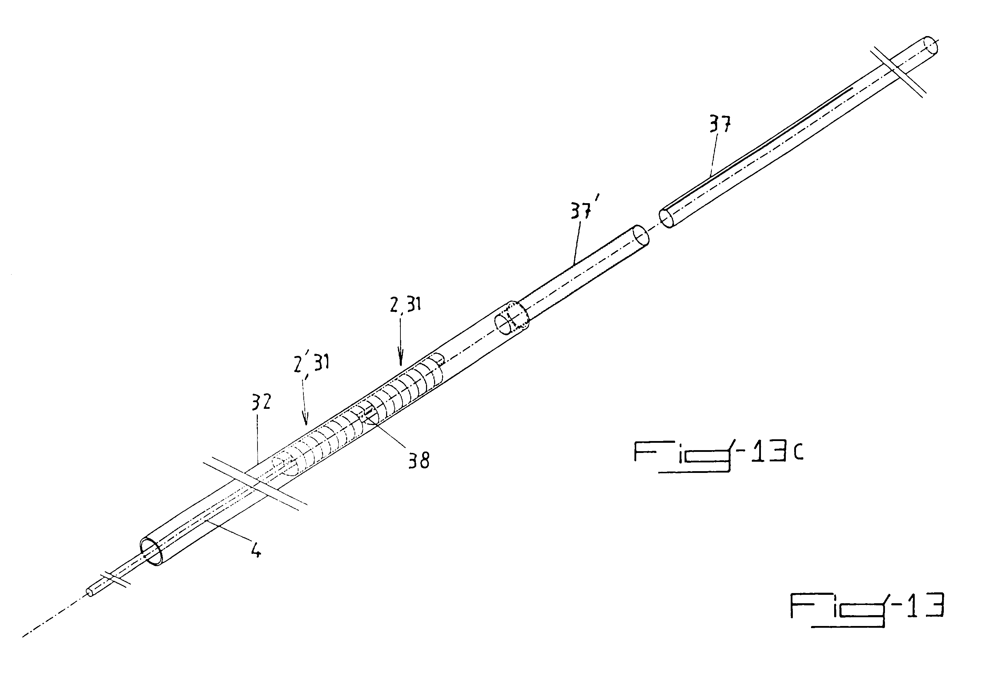

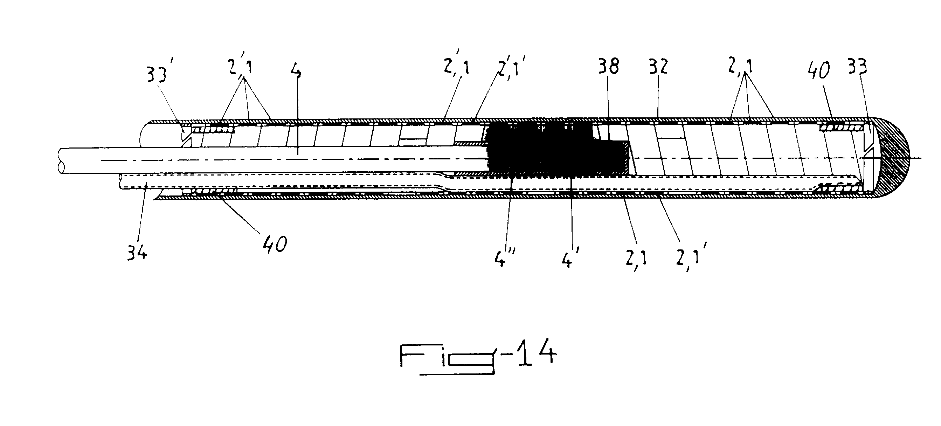

Comme le montrent les figures 1 et 10 des dessins annexés, la sonde comprend essentiellement d'une part, au moins une antenne 1 allongée formée par au moins une portion de conducteur 2, 2' à développement hélicoïdal rapportée sur une structure support diélectrique 3 et présentant une extrémité 3' avant et une extrémité arrière 3", d'autre part, des moyens de liaison électrique pour le transfert de signaux micro-ondes vers et à partir de ladite au moins une antenne 1, reliés à un générateur et à un radiomètre extérieurs correspondants, et, enfin, un cathéter 5 recouvrant ladite ou lesdites antenne(s) 1 et, le cas échéant, au moins la partie des moyens de liaison électrique 4 proximale de ladite ou desdites antenne(s) 1.

Conformément à l'invention, la structure support diélectrique 3 présente ou coopère avec des moyens 7, 7', 9, 10 ; 19, 24 ; 20, 28 ; 32, 34 de circulation de fluide de thermostatisation de l'antenne 1, 1' à l'intérieur du canal tubulaire délimité par ladite au moins une portion de conducteur hélicoïdale 2, 2', ces moyens étant reliés à des moyens 6, 6', 6", 6"' d'alimentation et d'évacuation dudit fluide de thermostatisation.

Grâce à cette disposition, il est possible de thermostatiser l'antenne I ou les antennes 1, 1', afin de minimiser et de contrôler son bruit thermique propre néfaste pour la mesure radiométrique et d'éviter une élévation de température trop importante de l'antenne 1 ou des antennes 1, 1' et de la ligne d'alimentation au cours de la phase d'émission, sans toutefois influencer la mesure radiométrique par un effet d'absorption.

En outre, il est également possible, en rapprochant au maximum l'antenne 1 des tissus à traiter, d'utiliser de manière optimale la zone de très forte intensité du champ électromagnétique haute fréquence rayonnée par l'antenne 1, et qui se situe à proximité de l'enveloppe extérieure de cette dernière, pour une nécrose rapide des tissus superficiels, y compris la paroi urétrale, permettant de supprimer l'effet de douleur par la destruction de la microcirculation et des terminaisons nerveuses situées à la surface desdits tissus.

En effet, le but de la mise en oeuvre de la sonde selon l'invention, en appliquant le protocole précité, est de créer une loge prostatique la plus profonde possible.

En effet, plus le diamètre de cette loge est important, meilleur est le résultat clinique. Il est bien connu de l'homme de l'art, spécialiste en micro-ondes et radiofréquences, que plus la fréquence augmente, plus la pénétration des ondes électromagnétiques est faible. C'est pourquoi les fréquences ISM (pour application scientifiques et médicales) élevées telles que par exemple 2450 MHz, ne sont pas utilisées pour l'application selon l'invention, bien que le coût de mise en oeuvre de cette fréquence soit faible à cause de son utilisation dans le domaine grand public (fours à micro-ondes). On cherche donc, dans le cadre de l'invention, à utiliser des fréquences basses telles que notamment 434 MHz, fréquence également autorisée, ce qui permet d'augmenter sensiblement la profondeur de pénétration et donc la profondeur de la loge prostatique.

De manière avantageuse, chaque antenne 1, 1' est formée par deux portions de conducteur hélicoïdales 2 et 2' sous forme de rubans métalliques plats, connectées au niveau de leurs extrémités adjacentes et présentant des sens d'enroulement opposés à partir desdites extrémités adjacentes, les moyens de liaison électrique 4 consistant en un câble coaxial, dont la connexion à un ensemble générateur micro-onde/radiomètre est réalisée à travers un câble de raccordement et par l'intermédiaire de connecteurs adaptés.

Dans le cas d'une sonde urétrale notamment, et en vue d'assurer de manière précise et reproductible son placement au niveau de la prostate, le cathéter 5 peut être pourvu, au niveau de son extrémité avant, de moyens 5' expansibles dans la vessie pour le positionnement de la partie active de la sonde dans la cavité ou le conduit (prostate) et d'un moyen 5" de positionnement et de blocage longitudinal de l'antenne 1 ou des antennes 1, 1' dans ledit cathéter 5.

Le moyen 5" pourra, par exemple, consister en un élément allongé rigide ou semi-rigide disposé dans la partie avant du cathéter 5 et faisant butée pour l'extrémité avant 3' de la structure support diélectrique 3 de manière à bloquer ce dernier, lors de l'introduction de l'antenne 1 dans le cathéter 5, dans une situation déterminée par rapport aux moyens expansibles 5'.

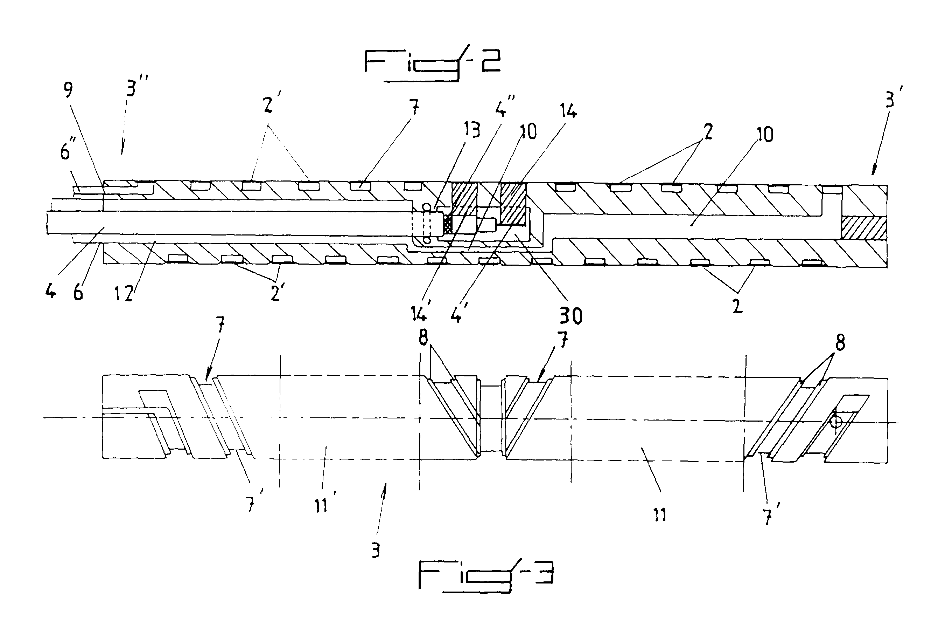

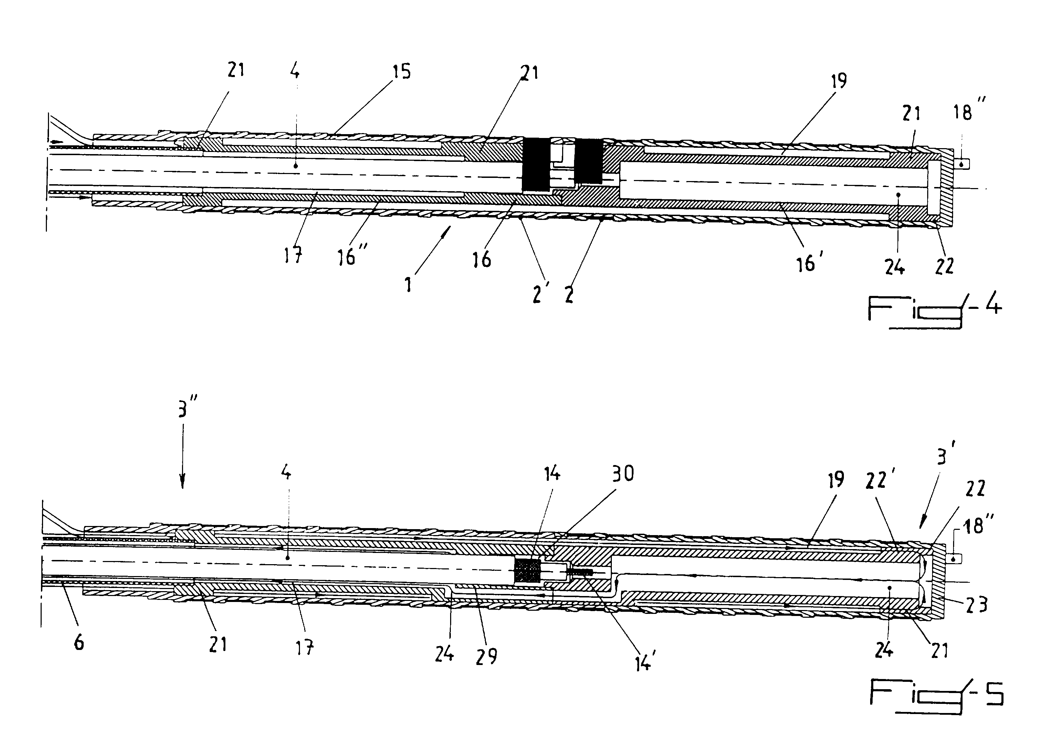

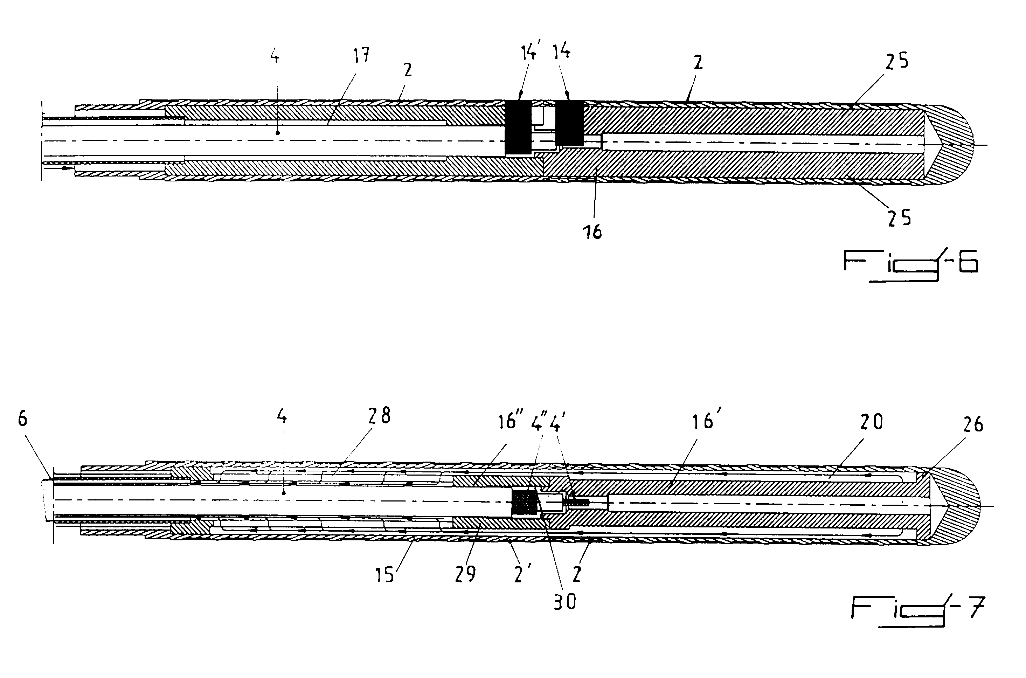

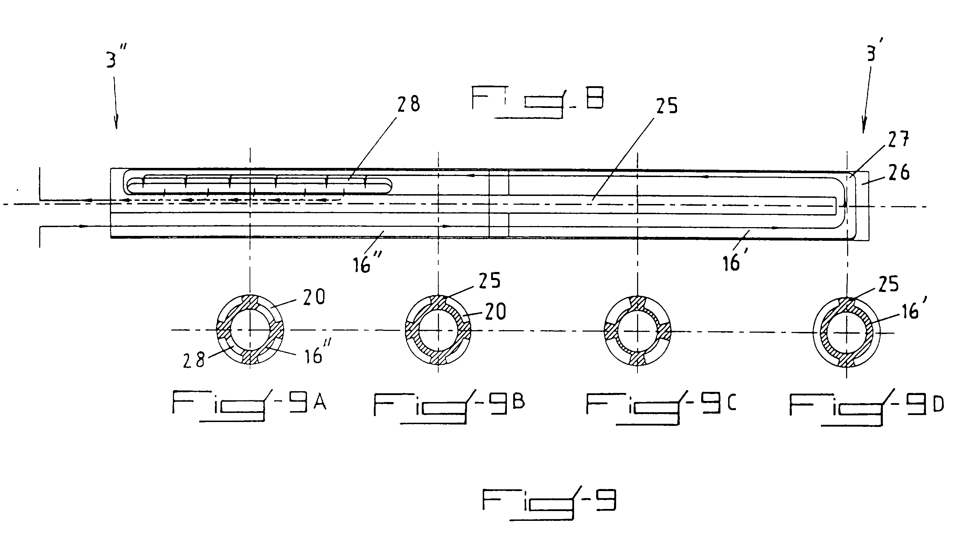

Ces derniers pourront consister, par exemple, en un ballonnet gonflable par injection d'un liquide ou d'un fluide de gonflage à travers un connecteur 18 (muni d'une valve anti-retour), un tuyau flexible 18' et un conduit ménagé dans la structure support diélectrique 3, ou un tuyau flexible disposé entre l'antenne et le cathéter, et débouchant, par un orifice ou un manchon 18", au niveau de la face frontale de l'extrémité avant 3' de la structure support 3, dans un canal 5"' ménagé dans l'élément allongé rigide 5" et communiquant avec l'intérieur dudit ballonnet 5' (voir figures 4 et 5).

Toutefois, les moyens expansibles 5' pourront également consister en un manchon logé dans l'extrémité avant du cathéter 5 et découpé en des lamelles longitudinales, ces lamelles pouvant être cintrées, avec extension radiale, pour former un corps en forme de sphère, par le déplacement d'un mandrin actionné par voie manuelle, hydraulique ou électrique.

La première variante de réalisation précitée conduit à une sonde du type Foley et la seconde à une sonde du type Malecot.

Pour obtenir une sonde de faible dimension radiale, permettant son introduction dans des conduits de faible section, toutes les portions de conducteur hélicoïdales 2, 2' formant l'antenne 1 ou les antennes 1, 1' sont enroulées sensiblement symétriquement autour d'un même axe longitudinal (voir les figures des dessins annexés) et reposent sensiblement sur une même surface cylindrique fictive, à section circulaire, formant le canal tubulaire à l'intérieur duquel circule le fluide de thermostatisation.

Afin de réduire le bruit thermique interne du câble coaxial 4 susceptible d'influencer la mesure radiométrique et de le fixer à une valeur précise connue et réglable, il peut être prévu que le fluide de thermostatisation circule à l'aller ou, préférentiellement, au retour, autour des moyens de liaison électrique 4 dans une gaine tubulaire 6 et en formant un manchon longitudinal enveloppant lesdits moyens de liaison électrique 4, notamment la partie de ces derniers pouvant être située dans la cavité ou le conduit du corps humain.

Cette gaine tubulaire 6 sera avantageusement reliée à un connecteur 6' pour le branchement à une conduite d'évacuation, la température du fluide de thermostatisation évacué étant mesurée par des moyens adéquats en vue du contrôle de la température de l'antenne 1 ou des antennes 1, 1' et du câble coaxial 4 et d'une correction adaptée de la mesure radiométrique de la température des tissus.

De manière analogue, les moyens d'alimentation 6", 6"' en fluide de thermostatisation peuvent consister en une gaine 6" entourant la gaine 6 et reliée à un connecteur 6"' de raccordement à une source de fluide de thermostatisation.

Toutefois, ledit moyen 6" d'alimentation en fluide de thermostatisation peut également se présenter sous la forme d'un tuyau flexible, ce en fonction de la forme des moyens de circulation de fluide dans le support diélectrique 3 (voir figure 2).

Selon une caractéristique de l'invention, et en vue de réduire les échanges calorifiques entre la sonde et les tissus environnants et de maintenir une différence de température importante entre lesdits tissus et l'antenne 1 ou les antennes 1, 1', favorable à une mesure radiométrique précise, ladite sonde peut avantageusement être recouverte sur sa face externe soit d'une couche, soit d'une gaine, notamment thermorétractable, ou analogue en un matériau thermiquement isolant.

En variante, il peut également être prévu que le cathéter 5 lui-même soit réalisé en un matériau thermiquement isolant.

Néanmoins, il est également souhaitable, pour les raisons évoquées précédemment, que l'antenne 1 soit située à proximité immédiate des tissus et pour ce faire le matériau isolant précité, par exemple soit du type connu sous la désignation polyéthylène, soit du type connu sous la désignation PTFE, est présent avec une épaisseur minimale, mais suffisante pour constituer une isolation thermique entre l'antenne 1 ou les antennes 1, 1' et la surface externe de la sonde, l'épaisseur étant notamment fonction de la différence de température recherchée entre l'antenne 1 ou les antennes 1, 1' et les tissus adjacents à celle-ci.

Selon une première variante de réalisation de l'invention, la sonde ne comporte qu'une unique antenne 1 fonctionnant à l'émission et à la réception.

Toutefois, selon une seconde variante de réalisation préférentielle de l'invention, la sonde comporte avantageusement deux antennes 1, 1' formées chacune par deux portions de conducteur hélicoïdales 2, 2' et reliées aux conducteurs du câble coaxial 4 formant moyens de liaison électrique, la première antenne 1 étant adaptée à la fréquence d'émission du générateur de micro-ondes et la seconde antenne 1' étant adaptée à la fréquence de réception du radiomètre, chacune desdites antennes 1, 1' présentant une impédance élevée à la fréquence d'adaptation de l'autre desdites antennes 1, 1'.

Ainsi, les actions et les effets de chacune des antennes 1 et 1' seront négligeables à la fréquence ou dans la plage de fréquences de fonctionnement de l'autre desdites antennes 1, 1'.

Afin de réduire l'encombrement de l'ensemble antennes 1, 1'/ corps support diélectrique 3 et de disposer les portions de conducteurs 2, 2' de chacune desdites antennes 1 et 1' à une distance minimale des tissus à traiter, les spires des portions de conducteur hélicoïdales 2, 2' formant l'antenne 1' adaptée à la réception radiométrique, sont disposées entre les spires des portions de conducteur hélicoïdales 2, 2' formant l'antenne 1 adaptée en émission, les extrémités des portions de conducteur hélicoïdales 2, 2' de l'antenne 1' s'amincissant de manière progressive, ce en vue d'éviter les pointes de courant pouvant résulter d'une coupure brutale des portions de conducteur hélicoïdales formant l'antenne 1' de plus faible dimension.

Le fluide de thermostatisation circulant dans la structure support diélectrique 3, et qui consiste préférentiellement en de l'eau, peut également servir à une bonne adaptation de l'antenne 1 avec le générateur de micro-ondes, qui émet notamment des fréquences de 915 MHz ou, préférentiellement, de 434 MHz environ, et avec le radiomètre, qui est adapté pour traiter plus particulièrement des fréquences de signaux de bruit thermique comprises ente 2 GHz et 4 GHz.

Il en est de même, bien entendu, en ce qui concerne le rôle de l'eau dans l'adaptation de l'antenne 1' à la plage de fréquences de la mesure radiométrique.

A cet effet, la quantité de fluide de thermostatisation, notamment de l'eau, présent dans le canal cylindrique formé par les portions de conducteur hélicoïdales 2 et 2' est suffisante pour limiter la longueur desdites portions de conducteur 2 et 2' à une longueur axiale (du canal tubulaire formé par ces portions de conducteur) inférieure à 8 cm, préférentiellement inférieure à 6 cm (notamment en ce qui concerne l'antenne 1), tout en autorisant une adaptation d'impédance optimale des antennes 1 et 1' aux fréquences de fonctionnement souhaitées du générateur de micro-ondes et du radiomètre (non représentés).

En plus de l'effet de thermostatisation, l'eau présente à l'intérieur de l'antenne 1 a également pour effet de raccourcir la longueur électrique de cette dernière de telle manière que sa longueur, notamment pour une fréquence de fonctionnement d'environ 434 MHz, ne soit pas plus importante que la longueur de l'urètre située dans la prostate.

En effet, l'élément rayonnant conçu pour transférer le maximum d'énergie à la prostate à 434 MHz (adaptation d'impédance) aurait dans l'air une longueur de 17 cm ce qui n'est pas compatible avec la longueur de l'urètre prostatique. La présence du fluide de thermostatisation permet de réduire la longueur de l'antenne à 5 cm environ tout en conservant une adaptation d'impédance optimale.

Conformément à un premier mode de réalisation de l'invention, représenté aux figures 2 et 3 des dessins annexés, le fluide de thermostatisation circule, au niveau de l'antenne 1 ou des antennes 1, 1', au moins en partie, selon un trajet parallèle et adjacent aux portions de conducteur hélicoïdales 2 et 2' (avec interposition d'une couche étanche diélectrique), en suivant le développement de ces dernières et en étant toujours situé du côté desdites portions 2 et 2' opposé à la surface externe de la sonde.

Plus précisément, la structure support diélectrique consiste en un corps allongé 3 de forme cylindrique et peut présenter sur sa surface extérieure une rainure 7 à développement hélicoïdal pourvue sur ses deux faces latérales, à proximité de la surface extérieure dudit support 3, d'épaulements continus en regard 8, ladite rainure 7 étant reliée, d'une part, au niveau de son extrémité proche de l'extrémité arrière 3" du support 3, à une conduite 9 d'alimentation en fluide de thermostatisation et, d'autre part, au niveau de son extrémité proche de l'extrémité avant 3' du support 3, à une conduite 10 d'évacuation de fluide de thermostatisation ménagée longitudinalement dans ledit support 3 et débouchant dans une gaine tubulaire 6 entourant les moyens de liaison électrique 4.

La rainure 7, qui consiste en deux ou quatre portions de rainure à développements opposés à partir de la zone médiane du support diélectrique 3, reçoit les portions de conducteur 2 et 2' sous forme de rubans au niveau des épaulements 8, sur lesquels lesdites portions 2 et 2' peuvent être solidarisées par collage de leur face inférieure recouverte d'un matériau isolant adapté.

Ainsi, lesdites portions de conducteur 2 et 2' forment ensemble avec une gorge centrale 7' de la rainure 7, située entre les épaulements 8, un canal de circulation de fluide de thermostatisation directement adjacent auxdites portions 2 et 2'.

Selon une caractéristique de l'invention, la structure support diélectrique 3 est préférentiellement constitué par deux moitiés de support 11, 11' assemblées coaxialement par emboîtement partiel et collage au niveau de la région médiane de l'antenne 1 ou des antennes 1 et 1', à savoir, d'une part, une première moitié de support 11 portant la ou les première(s) portion(s) de conducteur hélicoïdale(s) 2 et présentant une perforation longitudinale axiale 12 pour la réception de la portion avant du câble coaxial 4 formant les moyens de liaison électrique, ainsi qu'une bride 13 annulaire de maintien et de blocage de l'extrémité avant de ladite portion du câble coaxial 4 et, d'autre part, une seconde moitié de support 11' portant la ou les seconde(s) portion(s) de conducteur hélicoïdale(s) 2', lesdites moitiés de support 11, 11' étant pourvues, à proximité de leurs zones d'assemblage, de passages radiaux 14, 14' sous forme de fentes pour le passage des extrémités des portions de conducteur hélicoïdales 2, 2' en vue de leur connexion, respectivement, avec le conducteur filaire central 4' et le conducteur tubulaire externe 4" du câble coaxial 4.

Cette perforation à extension axiale 12 ménage un interstice en forme de manchon autour du câble coaxial 4 pour la circulation du fluide de thermostatisation entre la conduite 10 et le manchon 6, ce dernier étant relié à ladite perforation axiale 12.

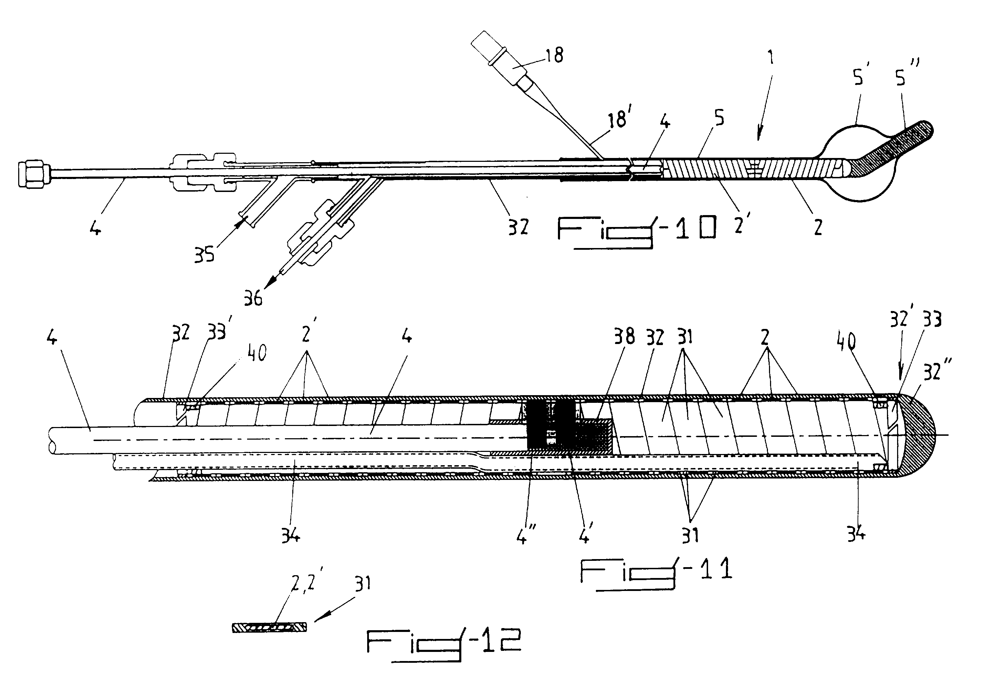

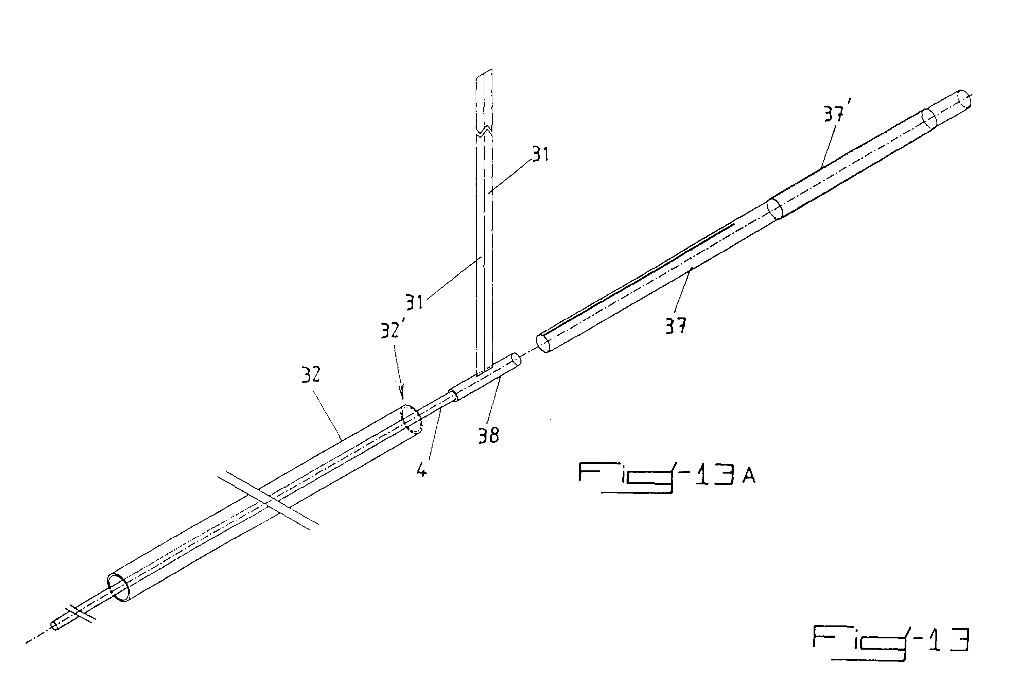

Comme le montrent les figures 4 à 9 des dessins annexés et conformément à un deuxième mode de réalisation de l'invention, le support diélectrique allongé 3 est constitué par un manchon extérieur 15 monté sur un mandrin intérieur 16, le manchon extérieur 15 portant les portions de conducteur hélicoïdales 2 et 2' et mandrin intérieur 16, présentant dans sa partie arrière une perforation axiale allongée 17 recevant la portion avant du câble coaxial 4 formant les moyens de liaison électrique et relié à une gaine tubulaire 6 entourant ledit câble coaxial 4.

Le mandrin intérieur 16 est monté dans le manchon extérieur 15 avec prévision d'un interstice annulaire longitudinal 19 ou de portions d'interstice annulaire longitudinaux 20 entre ces deux manchons 15 et 16, le fluide de thermostatisation circulant dans cet interstice 19 ou ces portions d'interstice 20.

Selon une première variante du deuxième mode de réalisation de l'invention, représentée aux figures 4 et 5 des dessins annexés, le mandrin intérieur 16 est, d'une part, pourvu, sur sa face externe, et au moins au niveau de ses extrémités longitudinales, de portions saillantes radiales 21 limitées en longueur, disposées avec un espacement régulier autour desdites extrémités et prenant appui sur la face interne du manchon extérieur 15 après assemblage par emboîtement dudit mandrin 16 dans ledit manchon 15 et, d'autre part, muni de portions saillantes frontales 22 au niveau de l'extrémité avant dudit mandrin 16, prenant appui, après assemblage, sur une paroi de fermeture 23 de l'extrémité avant du manchon extérieur 15 et ménageant des passages de communication 22' entre l'interstice annulaire 19 existant entre le mandrin 16 et le manchon 15 et une conduite 24 d'évacuation de fluide de thermostatisation s'étendant longitudinalement dans ledit mandrin intérieur 16, débouchant sur la face frontale avant du mandrin 16 et relié à la perforation axiale 17 recevant la portion avant du câble coaxial 4.

Le fluide de thermostatisation est, dans cette variante de réalisation, injecté entre les portions saillantes 21 situées au niveau de l'extrémité arrière du mandrin 16 au moyen de la gaine 6".

Selon une seconde variante du deuxième mode de réalisation de l'invention, représentée aux figures 6 à 9 des dessins annexés, le mandrin intérieur 16 est pourvu, sur sa face externe, de portions saillantes 25 continues sur toute la longueur dudit mandrin 16 et délimitant entre elles des portions 20 d'interstice annulaire formant des canaux séparés de circulation de fluide de thermostatisation à section en portion d'anneau, certaines desdites portions saillantes 25 s'arrêtant à distance d'une paroi 26 de délimitation et de fermeture frontale avant desdits canaux, de manière à ménager, pour chaque canal, un passage 27 entre le canal considéré et au moins un canal voisin, au niveau de l'extrémité avant du manchon intérieur 16.

En vue de l'évacuation du fluide de thermostatisation, certaines des portions d'interstice annulaire 20 communiquent, à proximité de leurs extrémités arrière, avec la perforation axiale 17 recevant la portion avant du câble coaxial 4 par l'intermédiaire d'ouvertures radiales 28, notamment sous forme de trous oblongs ou de fentes.

Ainsi, le fluide de thermostatisation est injecté dans les portions d'interstice annulaire 20 dépourvues d'ouvertures radiales 28 et ouvertes sur l'extrémité arrière 3" du support 3, circule vers l'extrémité avant 3' jusqu'à la paroi 26, est ensuite injecté par les passages 27 dans les portions d'interstice annulaire 20 pourvues d'ouvertures radiales 28 et fermées au niveau de l'extrémité arrière 3" du support 3 et circule dans ces portions d'interstice annulaire 20 pour s'écouler à travers lesdites ouvertures radiales 28 dans la perforation axiale 17.

Selon une caractéristique de l'invention, applicable aux deux variantes de réalisation décrites ci-dessus, le mandrin intérieur 16 peut être avantageusement composé de deux moitiés de mandrin 16' et 16", assemblées coaxialement par emboîtement partiel et collage au niveau de la région médiane du support 3, la moitié 16" formant la partie arrière dudit mandrin 16 comportant ladite perforation axiale 17 pour la réception de la portion avant du câble coaxial 4 et une bride annulaire 29 pour le maintien et le blocage de l'extrémité avant du câble coaxial 4, lesdites moitiés de mandrin 16' et 16" étant pourvues, à proximité de leurs zones d'assemblage, de passages radiaux sous forme de fentes 14, 14' coïncidants avec des passages du manchon extérieur 15, pour le passage des extrémités des portions de conducteur hélicoïdales 2 et 2' en vue de leur connexion, respectivement, avec le conducteur filaire central 4' et le conducteur tubulaire externe 4" du câble coaxial 4.

L'assemblage des deux moitiés de support 11, 11' ou des deux moitiés de mandrin 16', 16" est bien entendu réalisé de telle manière que les rainures, les passages ou les conduits traversant les deux moitiés soient continus.

En outre, comme le montrent les figures 2, 4, 5, 6 et 7, il peut être prévu, dans chaque moitié de support 11, 11' ou de mandrin 16', 16" et au niveau des zones d'assemblage, des évidements axiaux formant, après assemblage, une chambre 30, délimitée d'un côté par la bride 13 ou 29 et pouvant être remplie, en vue de son étanchéification, d'une résine ou d'un matériau analogue.

Cette chambre étanche 30 pourra servir au branchement des extrémités des portions de conducteur 2 et 2' de l'antenne 1 ou des antennes 1, 1' sur les conducteurs interne 4' et externe 4" du câble coaxial, en assurant une isolation électrique fiable.

Selon une caractéristique de l'invention, les portions de conducteur hélicoïdales 2 et 2', notamment en cuivre, peuvent être rapportées par collage dans des rainures de réception correspondantes.

En variante, les portions de conducteur hélicoïdales 2 et 2' peuvent également être réalisées par dépôt d'une couche métallique, notamment du cuivre, suivi d'une gravure ou d'une érosion sélective formant les motifs hélicoïdaux constituant lesdites portions de conducteur.

Conformément à un troisième mode de réalisation de l'invention, représenté aux figures 10 à 13 des dessins annexés, chaque antenne 1, 1' est formée, ensemble avec la structure support diélectrique 3, par enroulement hélicoïdal d'au moins une portion de bande 31 en un matériau diélectrique flexible et comprenant, dans sa masse, une bande de matériau conducteur 2, 2', ladite au moins une portion de bande 31 étant appliquée contre la paroi interne de l'extrémité avant 32' d'un tube 32 en un matériau synthétique souple ou semi-rigide, ce sous l'action de la poussée élastique résultant d'un état de compression radiale résiduel de ladite au moins une portion de bande 31 dans ledit tube 32.

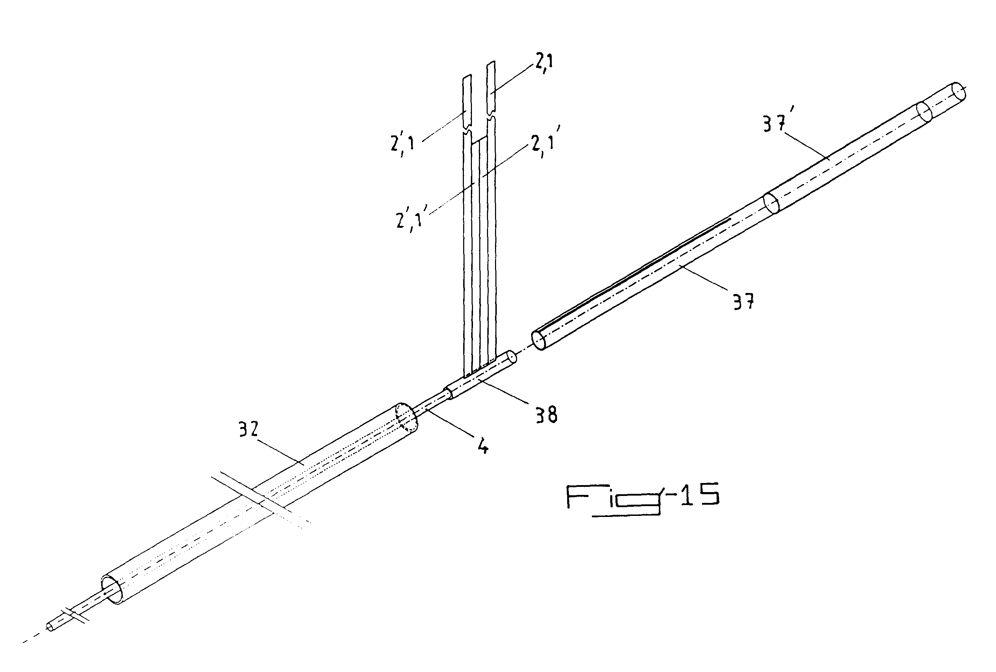

Comme le montre plus précisément la figure 12 des dessins annexés, la bande 31 comprend une bande conductrice centrale 2, 2' prise en sandwich, de manière étanche, entre deux couches de matériau diélectrique tel que, par exemple, du polyamide (par exemple du type connu sous la désignation CAPTON - marque déposée) ou du polyester (par exemple du type connu sous la désignation MYLAR - marque déposée).

Cette bande 31 pourra avantageusement être découpée dans un circuit imprimé souple comprenant plusieurs bandes en parallèle et utilisé normalement en tant que limande de raccordement.

Comme le montre plus précisément la figure 11 des dessins annexés, l'antenne 1 est avantageusement constituée par enroulement, selon des sens opposés, de deux portions de bande 31, les spires des portions de bande 31 étant jointives et des butées radiales 33, 33', par exemple élastiques et sous forme annulaire, étant disposées au niveau des extrémités avant 3' et arrière 3" des portions de bande 31 formant la structure support diélectrique 3 et l'antenne 1.

Dans le cas d'une sonde à deux antennes 1 et 1', ces dernières sont constituées par enroulement, deux par deux et selon des sens opposés, de quatre portions de bande 31, chaque paire de portions de bande 31 comprenant une portion plus longue et une portion plus courte, les spires des paires des portions de bande 31 étant jointives et en appui au niveau des extrémités avant 3' arrière 3" contre des butées radiales 33, 33' (figure 14).

Avantageusement, les portions de bande 31 de plus faible longueur, destinées à former l'antenne 1', adaptée à la réception radiométrique, s'amincissent progressivement en direction de leurs extrémités libres.

La mise en place de butées axiales avant 33 et arrière 33' permet, d'une part, de bloquer longitudinalement les deux ou les quatre enroulements hélicoïdaux des portions de bande 31 formant l'antenne 1 et les antennes 1, 1' et, d'autre part, de maintenir la compression longitudinale desdits enroulements dans le tube 32.

La connexion électrique entre les portions de conducteurs 2, 2' des enroulements hélicoïdaux de portions de bande 31 et les conducteurs du câble coaxial 4, formant moyens de liaison électrique, est obtenue en dénudant les extrémités adjacentes des deux portions de bandes 31 précitées et en les reliant aux conducteurs dudit câble coaxial 4, la zone de branchement et les parties dénudées des portions de bande 31 et du câble coaxial 4 étant enfermées dans une gaine ou un corps isolant étanche 38, rempli(e), le cas échéant, de résine ou d'un matériau isolant analogue.

La circulation du fluide de thermostatisation est assurée, d'une part, par le tube 32, dont l'extrémité avant est obturée de manière étanche et dont le prolongement arrière, contenant le câble coaxial 4, est relié à un dispositif 35 d'injection de fluide de thermostatisation, et, d'autre part, par un conduit tubulaire 34 disposé dans le tube 32 et débouchant au niveau de l'extrémité avant de ce dernier, ledit conduit tubulaire 34 étant relié à un dispositif 36 d'évacuation ou de recirculation du fluide de thermostatisation.

Le fluide de thermostatisation pourra, par exemple, être injecté librement, par écoulement, au moyen du tube 32 et être aspiré, après avoir circulé le long du câble coaxial 4 et à l'intérieur du canal tubulaire délimité par les portions de bande 31, par le conduit 34 au niveau de l'extrémité avant 32' dudit tube 32.

Ce troisième mode de réalisation selon l'invention, permet d'obtenir une sonde entièrement souple et déformable, du fait de la structure non rigide de chaque antenne 1, 1' et d'aboutir à un volume maximal de fluide de thermostatisation à l'intérieur de l'antenne 1 ou des antennes 1 et 1'.

Comme le montrent les figures 13A à 13C des dessins annexés, la réalisation d'une telle sonde souple comprenant une antenne 1, peut consister par exemple, à opérer les liaisons électrique entre le câble coaxial 4 et les portions de conducteurs 2, 2' des deux portions de bande 31, à introduire le câble coaxial 4 dans un tube 32 et l'extrémité avant dudit câble 4 reliée aux portions de bande 31 dans un mandrin creux fendu 37, dont le diamètre extérieur est inférieur au diamètre intérieur du tube 32 (figure 13A), à enrouler de manière serrée lesdites portions de bande 31 sur ledit mandrin 37 en bloquant les deux extrémités des deux portions de bande 31 dans la ou les fentes dudit mandrin 37, puis à enmancher le tube 32 sur l'extrémité avant du câble coaxial 4 et sur la portion du mandrin 37 portant les portions de bande 31 jusqu'au contact dudit mandrin 37 avec la butée arrière 33' prémontée dans le tube 32 (figure 13B), à extraire ensuite le mandrin 37 du tube 32 en retenant les portions de bande 31 dans ledit tube 32 au moyen d'un manchon repoussoir 37' (au moment du retrait du mandrin 37, les portions de bande 31 se détendent partiellement et s'appliquent intérieurement contre le tube 32 - Figure 13C), à mettre en place la butée avant 33, le cas échéant, en comprimant longitudinalement les portions de bande 31 à l'aide du mandrin 37, et, enfin, à obturer l'extrémité avant 32' du tube 32, par exemple en ramollissant le matériau dudit tube 32 de manière à pouvoir former un bouchon 32".

La réalisation d'une sonde du type précité comprenant deux antennes 1 et 1' peut comprendre les mêmes étapes que celles décrites ci-dessus, sauf à utiliser deux paires de portions de bande 31 de longueurs différentes (figure 15).

En vue d'ajuster et de régler la configuration du champ rayonné, il peut être, selon une caractéristique complémentaire de l'invention, prévu que la sonde comporte, au moins au niveau des extrémités distales opposées de l'antenne 1 ou des antennes 1, 1', des pièces supplémentaires ou des inserts 40 en un matériau diélectrique, par exemple en forme de bagues, disposé(e)s dans le canal tubulaire délimité par ladite au moins une portion de bande 31 réalisant l'antenne 1 ou les antennes 1, 1'.

Ainsi, il est possible, en agissant sur les différentes caractéristiques de ces pièces supplémentaires rapportées ou inserts 40, à savoir notamment leur longueur, leur épaisseur ou largeur, la nature du matériau les constituant (valeur de la permitivité) et leurs emplacements, de construire des sondes adaptées spécifiquement à un volume à traiter donné.

En leur conférant avantageusement une forme en bague ou en anneau, on ne gène pratiquement pas la libre circulation du fluide de thermostatisation dans le canal tubulaire formé par l'antenne 1 ou les antennes 1 et 1', on conserve la souplesse de la sonde (utilisation d'un matériau diélectrique préférentiellement souple) et on autorise une mise en place aisée et un positionnement sûr et définitif desdites pièces ou inserts 40 dans ladite sonde (diamètre extérieur déterminé pour un maintien avec serrage dans le canal tubulaire), au moment de la réalisation de cette dernière.

Grâce à l'invention, il est possible de mettre en oeuvre de nouvelles techniques de thermothérapie basées sur des opérations de chauffage des tissus plus performantes en termes de montée en température et en valeur de température moyenne de traitement.

En effet, la technique classique de thermothérapie dans le domaine de l'adénome prostatique consiste à chauffer les tissus prostatiques à des températures modérées de l'ordre de 45° C et en ne dépassant pas 50 ° C dans la prostate et les tissus superficiels.

On atteint ainsi dans les tissus prostatiques des suffusions hémorragiques superficielles et l'utilisation de ces températures nécessite une séance de traitement de 60 minutes ou plus.

Les antennes utilisées sont des antennes de type filaire présentant un lobe de rayonnement concentré autour de sa partie médiane dans le sens longitudinal, et un rayonnement limité vers ses extrémités.

En particulier, le rayonnement est limité, donc le chauffage moindre, au niveau du col vésical où, selon les spécialistes urologues, il est nécessaire au contraire de s'assurer d'un chauffage suffisant afin d'agir également à ce niveau.

Le but de l'invention est donc de pouvoir chauffer de façon homogène depuis le col vésical jusqu'à la base de la prostate, de nécroser les tissus superficiels en incluant la paroi urétrale, d'accroître la profondeur de pénétration afin de créer une loge prostatique similaire à celle obtenue par résection endoscopique, tout en maintenant une température élevée à la surface.

De plus, le traitement doit être le plus court possible, sans nécessiter d'anesthésie.

Il est bien évident que le contrôle de température intraprostatique qui était nécessaire dans l'art antérieur devient primordial dans cette technique à haute température afin d'assurer l'efficacité du traitement et la sécurité du patient.

L'utilisation de la mesure de température "in situ" par radiométrie est donc impérative, car elle évite l'utilisation de capteurs locaux, c'est-à-dire de capteurs qui n'indiquent que des températures superficielles ou ponctuelles dans la prostate. De plus, ces capteurs locaux doivent être introduits dans la prostate et induisent par conséquent un traumatisme pour le patient.

Ainsi, grâce à l'invention, la solution mise en oeuvre est la suivante :
- Utilisation d'une antenne à lignes à retard rayonnantes de formes hélicoïdales (demande de brevet français n° 2 711 066) présentant un lobe de rayonnement longitudinal plus homogène par rapport à l'antenne filaire et permettant de traiter également le col vésical ;
- Diminution de la fréquence de travail, préférentiellement à 434 MHz, afin d'accroître la profondeur de pénétration du chauffage (dans le cas de petites prostates, ou selon la nature des tissus, on pourra éventuellement travailler à la fréquence de 915 MHz) ;
- Obtention d'une température intra-prostatique de l'ordre de 60° C au cours du traitement ;
- Montée en température très rapide, notamment en un intervalle de temps inférieur à 5 minutes, afin de détruire rapidement la micro-circulation sanguine, les terminaisons nerveuses et la paroi urétrale ;
- Contrôle de la température intra-prostatique par la technique radiométrique.

Par rapport à l'utilisation d'une antenne à lignes à retard rayonnantes classique, l'obtention des résultats précités a nécessité la résolution des points suivants par l'invention :
- S'assurer que les tissus superficiels sont détruits, donc que l'antenne se trouve le plus près possible de la paroi urétrale ;
- S'assurer que la température mesurée par voie radiométrique est la plus fiable possible, d'une part, en :
   * thermostatisant l'antenne et le câble de connexion de l'antenne par circulation de fluide afin de ne pas prendre en compte le bruit thermique propre de l'antenne et du câble et, d'autre part, en
   * supprimant la prise en compte de la mesure de température du fluide de thermostatisation dans la mesure radiométrique.
- Réduire la longueur de ces éléments rayonnants par l'adjonction d'un diélectrique présentant un εᵣ élevé (en effet, la longueur d'un élément rayonnant dans l'air à 915 MHz est de 8 cm et à 434 MHz celle-ci est de 17 cm, ces longueurs étant incompatibles avec les tailles de prostate qui ne dépassent pratiquement jamais 5 cm).
- Limiter les échanges thermiques par conduction thermique entre la prostate et le fluide de thermostatisation.

En pratique, le choix du diélectrique se porte sur l'eau qui présente des pertes diélectriques relativement élevées (échauffement important, donc circulation de l'eau impérative) mais également une valeur de constante diélectrique élevée (de l'ordre de 80) aux fréquences utilisées.

Le problème complexe résolu par l'invention consiste donc en l'utilisation d'une circulation de fluide de thermostatisation de l'antenne et du câble de connexion afin de fiabiliser la mesure radiométrique et diminuer la longueur de l'antenne dans le cas de l'utilisation de la fréquence 434 MHz, tout en plaçant l'antenne le plus près possible de la paroi urétrale afin d'éviter la mesure de température du fluide de thermostatisation par le radiomètre et de délivrer le maximum d'énergie dans la prostate pour l'obtention d'une nécrose superficielle et profonde des tissus prostatiques.

Bien entendu, l'invention n'est pas limitée aux modes de réalisation décrits et représentés aux dessins annexés. Des modifications restent possibles, notamment du point de vue de la constitution des divers éléments ou par substitution d'équivalents techniques, sans sortir pour autant du domaine de protection de l'invention.

## Revendications

1. Sonde pour le chauffage de tissus par micro-ondes et pour la mesure de température par radiométrie micro-onde, destinée à être introduite dans une cavité ou un conduit du corps humain, permettant la destruction des tissus environnants, notamment des tissus prostatiques et de la paroi urétrale, et comprenant essentiellement, d'une part, au moins une antenne allongé (1,1') formée par au moins une portion de conducteur (2,2') à développement hélicoïdal rapportée sur une structure support diélectrique (3) et présentant une extrémité avant (3') et une extrémité arrière (3"), d'autre part, des moyens de liaison électrique (4) pour le transfert de signaux micro-ondes vers et à partir de ladite au moins une antenne (1,1'), susceptibles d'être connectés à un générateur et à un radiomètre extérieurs correspondants, et, enfin, un cathéter (5) recouvrant ladite ou lesdites antenne(s) (1,1') et, le cas échéant, au moins la partie des moyens de liaison électrique (4) proximale de ladite ou desdites antenne(s) (1,1'), la structure support diélectrique (3) présentant des moyens de circulation de fluide de thermostatisation de l'antenne, ces moyens de circulation étant reliés à des moyens d'alimentation et d'évacuation dudit fluide de thermostatisation, sonde caractérisée en ce que lesdits moyens (7, 7', 9, 10 ; 19, 24 ; 20, 28 ; 32, 34) de circulation de fluide de thermostatisation sont adaptés pour que ledit fluide puisse circuler au niveau de l'antenne ou des antennes (1, 1') à l'intérieur du canal tubulaire délimité par ladite au moins une portion de conducteur hélicoïdale (2, 2'), en étant toujours situé du côté de ladite ou desdites portion(s) (2, 2') opposé à la surface externe de la sonde.

2. Sonde selon la revendication 1, caractérisée en ce que chaque antenne (1, 1') est formée par deux portions de conducteur hélicoïdales (2 et 2') sous forme de rubans métalliques plats, connectées au niveau de leurs extrémités adjacentes et présentant des sens d'enroulement opposés à partir desdites extrémités adjacentes, les moyens de liaison électrique (4) consistant en un câble coaxial.

3. Sonde selon l'une quelconque des revendications 1 et 2, caractérisée en ce que l'antenne (1) ou les antennes (1, 1') est recouverte sur sa face externe d'une couche ou d'une gaine, notamment thermorétractable, en un matériau thermiquement isolant.

4. Sonde selon l'une quelconque des revendications 1 et 2, caractérisée en ce que le cathéter (5) est réalisé en un matériau thermiquement isolant.

5. Sonde selon l'une quelconque des revendications 1 à 4, caractérisée en que le cathéter (5) est pourvu, au niveau de son extrémité avant, de moyens expansibles (5') de positionnement de la sonde dans la cavité ou le conduit et d'un moyen (5") de positionnement et de blocage longitudinal de l'antenne (1) ou des antennes (1, 1') dans ledit cathéter (5).

6. Sonde selon l'une quelconque des revendications 1 à 5, caractérisée en ce que le fluide de thermostatisation peut circuler, à l'aller ou au retour, autour des moyens de liaison électrique (4) dans une gaine tubulaire (6) et en formant un manchon longitudinal enveloppant lesdits moyens de liaison électrique (4), notamment la partie de ces derniers pouvant être située dans la cavité ou le conduit du corps humain.

7. Sonde selon l'une quelconque des revendications 1 à 6, caractérisée en ce qu'elle comporte deux antennes (1, 1') formées chacune par deux portions de conducteur hélicoïdales (2, 2') et reliées aux conducteurs du câble coaxial (4) formant moyens de liaison électrique, la première antenne (1) étant adaptée à la fréquence d'émission du générateur de micro-ondes et la seconde antenne (1') étant adaptée à la fréquence de réception du radiomètre, chacune desdites antennes (1, 1') présentant une impédance élevée à la fréquence d'adaptation de l'autre desdites antennes (1, 1').

8. Sonde selon la revendication 7, caractérisée en ce que les spires des portions de conducteur hélicoïdales (2, 2') formant l'antenne (1') adaptée à la réception radiométrique, sont disposées entre les spires des portions de conducteur hélicoïdales (2, 2') formant l'antenne (1) adaptée en émission, les extrémités des portions de conducteur hélicoïdales (2, 2') de l'antenne (1') s'amincissant de manière progressive.

9. Sonde selon l'une quelconque des revendications 1 à 8, caractérisée en ce que le fluide de thermostatisation peut circuler, au niveau de l'antenne (1) ou des antennes (1, 1'), au moins en partie, selon un trajet parallèle et adjacent aux portions de conducteur hélicoïdales (2 et 2').

10. Sonde selon la revendication 9, caractérisée en ce que la structure support diélectrique (3) consiste en un support allongé de forme cylindrique et présente sur sa surface extérieure une rainure (7) à développement hélicoïdal pourvue sur ses deux faces latérales, à proximité de la surface extérieure dudit support, d'épaulements continus en regard (8), ladite rainure (7) étant reliée, d'une part, au niveau de son extrémité proche de l'extrémité arrière (3") du support (3), à une conduite (9) d'alimentation en fluide de thermostatisation et, d'autre part, au niveau de son extrémité proche de l'extrémité avant (3') du support (3), à une conduite (10) d'évacuation de fluide de thermostatisation ménagée longitudinalement dans ledit support (3) et débouchant dans une gaine tubulaire (6) entourant les moyens de liaison électrique (4).

11. Sonde selon la revendication 10, caractérisée en ce que la structure support diélectrique (3) est constituée par deux moitiés de support (11, 11') assemblées coaxialement par emboîtement partiel et collage au niveau de la région médiane de l'antenne (1) ou des antennes (1, 1'), à savoir, d'une part, une première moitié de support (11) portant la ou les première(s) portion(s) de conducteur hélicoïdale(s) (2) et présentant une perforation longitudinale axiale (12) pour la réception de la portion avant du câble coaxial (4) formant les moyens de liaison électrique, ainsi qu'une bride (13) annulaire de maintien et de blocage de l'extrémité avant de ladite portion du câble coaxial (4) et, d'autre part, une seconde moitié de support (11') portant la ou les seconde(s) portion(s) de conducteur hélicoïdale(s) (2'), lesdites moitiés de support (11, 11') étant pourvues, à proximité de leurs zones d'assemblage, de passages radiaux (14, 14') sous forme de fentes pour le passage des extrémités des portions de conducteur hélicoïdales (2, 2') en vue de leur connexion, respectivement, avec le conducteur filaire central (4') et le conducteur tubulaire externe (4") du câble coaxial (4).

12. Sonde selon l'une quelconque des revendications 1 à 8, caractérisée en ce que le support diélectrique allongé (3) est constitué par un manchon extérieur (15) monté sur un mandrin intérieur (16), le manchon extérieur (15) portant les portions de conducteur hélicoïdales (2 et 2') et le mandrin intérieur (16), présentant dans sa partie arrière une perforation axiale allongée (17) recevant la portion avant du câble coaxial (4) formant les moyens de liaison électrique et relié à une gaine tubulaire (6) entourant ledit câble coaxial (4).

13. Sonde selon la revendication 12, caractérisée en ce que le mandrin intérieur (16) est monté dans le manchon extérieur (15) avec prévision d'un interstice annulaire longitudinal (19) ou de portions d'interstice annulaire longitudinaux (20) entre ces deux manchons (15 et 16), le fluide de thermostatisation circulant dans cet interstice (19) ou ces portions d'interstice (20).

14. Sonde selon la revendication 13, caractérisée en que le mandrin intérieur (16) est, d'une part, pourvu, sur sa face externe, et au moins au niveau de ses extrémités longitudinales, de portions saillantes radiales (21) limitées en longueur, disposées avec un espacement régulier autour desdites extrémités et prenant appui sur la face interne du manchon extérieur (15) après assemblage par emboîtement dudit mandrin (16) dans ledit manchon (15) et, d'autre part, muni de portions saillantes frontales (22) au niveau de l'extrémité avant dudit mandrin (16), prenant appui, après assemblage, sur une paroi de fermeture (23) de l'extrémité avant du manchon extérieur (15) et ménageant des passages de communication (22') entre l'interstice annulaire (19) existant entre le mandrin (16) et le manchon (15) et une conduite (24) d'évacuation de fluide de thermostatisation s'étendant longitudinalement dans ledit mandrin intérieur (16), débouchant sur la face frontale avant du mandrin (16) et relié à la perforation axiale (17) recevant la portion avant du câble coaxial (4).

15. Sonde selon la revendication 13, caractérisée en ce que le mandrin intérieur (16) est pourvu, sur sa face externe, de portions saillantes (25) continues sur toute la longueur dudit mandrin (16) et délimitant entre elles des portions d'interstice annulaire (20) formant des canaux séparés de circulation de fluide de thermostatisation à section en portion d'anneau, certaines desdites portions saillantes (25) s'arrêtant à distance d'une paroi (26) de délimitation et de fermeture frontale avant desdits canaux, de manière à ménager, pour chaque canal, un passage (27) entre le canal considéré et au moins un canal voisin, au niveau de l'extrémité avant du manchon intérieur (16).

16. Sonde selon la revendication 15, caractérisée en ce que certaines des portions d'interstice annulaire (20) communiquent, à proximité de leurs extrémités arrière, avec la perforation axiale (17) recevant la portion avant du câble coaxial (4) par l'intermédiaire d'ouvertures radiales (28), notamment sous forme de trous oblongs ou de fentes.

17. Sonde selon l'une quelconque des revendications 13 à 16, caractérisée en ce que le mandrin intérieur (16) est composé de deux moitiés de mandrin (16' et 16"), assemblées coaxialement par emboîtement partiel et collage au niveau de la région médiane du support (3), la moitié (16") formant la partie arrière dudit mandrin (16) comportant ladite perforation axiale (17) pour la réception de la portion avant du câble coaxial (4) et une bride annulaire (29) pour le maintien et le blocage de l'extrémité avant du câble coaxial (4), lesdites moitiés de mandrin (16' et 16") étant pourvues, à proximité de leurs zones d'assemblage, de passages radiaux sous forme de fentes (14, 14') coïncidants avec des passages du manchon extérieur (15), pour le passage des extrémités des portions de conducteur hélicoïdales (2 et 2') en vue de leur connexion, respectivement, avec le conducteur filaire central (4') et le conducteur tubulaire externe (4") du câble coaxial (4).

18. Sonde selon l'une quelconque des revendications 1 à 17, caractérisée en ce que les portions de conducteur hélicoïdales (2 et 2'), notamment en cuivre, sont rapportées par collage dans des rainures de réception correspondantes.

19. Sonde selon l'une quelconque des revendications 1 à 16, caractérisée en ce que les portions de conducteur hélicoïdales (2 et 2') sont réalisées par dépôt d'une couche métallique, notamment du cuivre, suivi d'une gravure ou d'une érosion sélective formant les motifs hélicoïdaux constituant lesdites portions de conducteur.

20. Sonde selon l'une quelconque des revendications 1 à 8, caractérisée en ce que chaque antenne (1, 1') est formée, ensemble avec la structure support diélectrique (3), par enroulement hélicoïdal d'au moins une portion de bande (31) en un matériau diélectrique flexible et comprenant, dans sa masse, une bande de matériau conducteur (2, 2'), ladite au moins une portion de bande (31) étant appliquée contre la paroi interne de l'extrémité avant (32') d'un tube (32) en un matériau synthétique souple ou semi-rigide, ce sous l'action de la poussée élastique résultant d'un état de compression radiale résiduel de ladite au moins une portion de bande (31) dans ledit tube (32).

21. Sonde selon la revendication 20, caractérisée en ce que l'antenne (1) est constituée par enroulement, selon des sens opposés, de deux portions de bande (31), les spires des portions de bande (31) étant jointives et des butées radiales (33, 33'), par exemple élastiques et sous forme annulaire, étant disposées au niveau des extrémités avant (3') et arrière (3") des portions de bande (31) formant la structure support diélectrique (3) et l'antenne (1).

22. Sonde selon la revendication 20, caractérisée en ce que les antennes (1 et 1') sont constituées par enroulement, deux par deux et selon des sens opposés, de quatre portions de bande (31), chaque paire de portions de bande (31) comprenant une portion plus longue et une portion plus courte, les spires des paires des portions de bande (31) étant jointives et en appui au niveau des extrémités avant (3') et arrière (3") contre des butées radiales (33, 33').

23. Sonde selon la revendication 22, caractérisée en ce que les portions de bande (31) de plus faible longueur, destinées à former l'antenne (1'), adaptée à la réception radiométrique, s'amincissent progressivement en direction de leurs extrémités libres.

24. Sonde selon l'une quelconque des revendications 20 ou 23 caractérisée en ce que la circulation du fluide de thermostatisation est assurée, d'une part, par le tube (32), dont l'extrémité avant est obturée de manière étanche et dont le prolongement arrière, contenant le câble coaxial (4), est relié à un dispositif (35) d'injection de fluide de thermostatisation, et, d'autre part, par un conduit tubulaire (34) disposé dans le tube (32) et débouchant au niveau de l'extrémité avant de ce dernier, ledit conduit tubulaire (34) étant relié à un dispositif (36) d'évacuation ou de recirculation du fluide de thermostatisation.

25. Sonde selon l'une quelconque des revendications 20 à 24, caractérisée en ce qu'elle comporte, au niveau des extrémités distales opposées de l'antenne (1) ou des antennes (1, 1'), des pièces supplémentaires ou des inserts (40) en un matériau diélectrique, par exemple en forme de bagues, disposé(e)s dans le canal tubulaire délimité par ladite au moins une portion de bande (31) réalisant l'antenne (1) ou les antennes (1, 1').

## Claims

1. A probe for heating tissues by microwaves and for temperature measurement by microwave radiometry, which is intended to be introduced into a cavity or a duct in the human body, permitting destruction of the surrounding tissues, in particular prostatic tissues and the urethral wall. and essentially comprising on the one hand at least one elongate antenna (1, 1') formed by at least one helicoidally disposed conductor portion (2, 2') fitted on to a dielectric support structure (3) and having a front end (3') and a rear end (3"), on the other hand electrical connection means (4) for the transfer of microwave signals towards and from said at least one antenna (1, 1'), which are capable of being connected to a corresponding external generator and radiometer, and finally a catheter (5) covering said antenna or antennae (1, 1') and if necessary at least the part of the electrical connection means (4) which is proximal to said antenna or antennae (1, 1'). the dielectric support structure (3) having means for the circulation of fluid for thermostatic control of the antenna, said circulation means being connected to means for the supply and discharge of said thermostatic control fluid, the probe being characterised in that said means (7, 7', 9, 10; 19, 24; 20, 28; 32, 34) for the circulation of fluid for thermostatic control are adapted for the fluid to be able to circulate at the location of the antenna or the antennae (1, 1') in the interior of the tubular passage delimited by said at least one helicoidal conductor portion (2. 2'), while always being disposed at the side of said portion or portions (2, 2') that is opposite to the outside surface of the probe.

2. A probe according to claim 1 characterised in that each antenna (1, 1') is formed by two helicoidal conductor portions (2 and 2') in the form of flat metal strips connected at the location of their adjacent ends and involving opposite directions of winding from said adjacent ends, the electrical connection means (4) consisting of a coaxial cable.

3. A probe according to either one of claims 1 and 2 characterised in that the antenna (1) or the antennae (1, 1') is or are covered on its outside face by a layer or a sheath, which in particular is heat-shrinkable, of a heat-insulating material.

4. A probe according to either one of claims 1 and 2 characterised in that the catheter (5) is made of a heat-insulating material.

5. A probe according to any one of claims 1 to 4 characterised in that at the location of its front end the catheter (5) is provided with expansible means (5') for positioning the probe in the cavity or the duct and a means (5") for longitudinal positioning and locking of the antenna (1) or the antennae (1, 1') in said catheter (5).

6. A probe according to any one of claims 1 to 5 characterised in that the thermostatic control fluid can flow, in the outward direction or in the return direction, around the electrical connection means (4) in a tubular sheath (6) forming a longitudinal casing enclosing said electrical connection means (4), in particular the part of the latter which can be disposed in the cavity or the duct of the human body.

7. A probe according to any one of claims 1 to 6 characterised in that it comprises two antennae (1, 1') each formed by two helicoidal conductor portions (2. 2') and connected to the conductors of the coaxial cable (4) forming electrical connection means, the first antenna (1) being matched to the emission frequency of the microwave generator and the second antenna (1') being matched to the reception frequency of the radiometer, each of said antennae (1, 1') having a high impedance at the matching frequency of the other of said antennae (1, 1').

8. A probe according to claim 7 characterised in that the turns of the helicoidal conductor portions (2, 2') forming the antenna (1') which is adapted for radiometric reception are disposed between the turns of the helicoidal conductor portions (2, 2') forming the antenna (1) adapted for emission, the ends of the helicoidal conductor portions (2, 2') of the antenna (1') becoming progressively thinner.

9. A probe according to any one of claims 1 to 8 characterised in that at the location of the antenna (1) or the antennae (1, 1') the thermostatic control fluid can flow at least in part along a path which is parallel and adjacent to the helicoidal conductor portions (2 and 2').

10. A probe according to claim 9 characterised in that the dielectric support structure (3) comprises an elongate support of cylindrical shape and has on its outside surface a groove (7) of helicoidal configuration provided on its two lateral faces in the proximity of the outside surface of the support with facing continuous shoulders (8), said groove (7) being connected on the one hand at the location of its end close to the rear end (3") of the support (3) to a conduit (9) for the supply of thermostatic control fluid and on the other hand at the location of its end close to the front end (3') of the support (3) to a conduit (10) for discharge of thermostatic control fluid disposed longitudinally in said support (3) and opening into a tubular sheath (6) surrounding the electrical connection means (4).

11. A probe according to claim 10 characterised in that the dielectric support structure (3) is formed by two support halves (11, 11') assembled coaxially by partial interengagement and glueing at the location of the central region of the antenna (1) or the antennae (1, 1'), namely on the one hand a first support half (11) bearing the first helicoidal conductor portion or portions (2) and having an axial longitudinal perforation (12) for receiving the front portion of the coaxial cable (4) forming the electrical connection means, and an annular collar (13) for holding and locking the front end of said portion of the coaxial cable (4), and on the other hand a second support half (11') bearing the second helicoidal conductor portion or portions (2'), said support halves (11, 11') being provided in the proximity of their assembly zones with radial passages (14, 14') in the form of slots for the passage of the ends of the helicoidal conductor portions (2, 2') for the purposes of connection thereof respectively to the central wire conductor (4') and the outside tubular conductor (4") of the coaxial cable (4).

12. A probe according to any one of claims 1 to 8 characterised in that the elongate dielectric support structure (3) is formed by an outside casing (15) mounted on an inside bar (16), the outside casing (15) bearing the helicoidal conductor portions (2 and 2') and the inside bar (16) having in its rear part an elongate axial perforation (17) receiving the front portion of the coaxial cable (4) forming the electrical connection means and connected to a tubular sheath (6) surrounding said coaxial cable (4).

13. A probe according to claim 12 characterised in that the inside bar (16) is mounted in the outside casing (15) with the provision of an annular longitudinal gap (19) or longitudinal annular gap portions (20) between said two casings (15 and 16), the thermostatic control fluid flowing in said gap (19) or said gap portions (20).

14. A probe according to claim 13 characterised in that the inside bar (16) is on the one hand provided on its outside face and at least at the level of its longitudinal ends with radial projecting portions (21) which are limited in respect of length and which are disposed at a regular spacing around said ends and bearing against the inside face of the outside casing (15) after assembly by interengagement of said bar (16) into said casing (15) and on the other hand provided with frontal projecting portions (22) at the level of the front end of said bar (16) bearing after assembly against a closure wall (23) of the front end of the outside casing (15) and providing communication passages (22') between the annular gap (19) existing between the bar (16) and the casing (15) and a conduit (22) for discharge of thermostatic control fluid extending longitudinally in said inside bar (16). opening at the front frontal face of the bar (16) and connected to the axial perforation (17) receiving the front portion of the coaxial cable (4).

15. A probe according to claim 13 characterised in that the inside bar (16) is provided on its outside face with projecting portions (25) which are continuous over the entire length of said bar (16) and delimiting between them annular gap portions (20) forming separate ducts for flow of thermostatic control fluid involving a section in the form of a portion of a ring, some of said projecting portions (25) stopping at a spacing from a wall (26) for delimitation and front frontal closure of said ducts so as to provide for each duct a passage (27) between the duct in question and at least an adjacent duct at the level of the front end of the inside bar (16).

16. A probe according to claim 15 characterised in that some of the annular gap portions (20) communicate in the proximity of the rear ends thereof with the axial perforation (17) receiving the front portion of the coaxial cable (4) by way of radial openings (28), in particular in the form of oblong holes or slots.

17. A probe according to any one of claims 13 to 16 characterised in that the inside bar (16) is composed of two bar halves (16' and 16") which are assembled in coaxial relationship by partial interengagement and glueing at the location of the central region of the suoport (3), the half (16") forming the rear part of said bar (16) comprising said axial perforation (17) for receiving the front portion of the coaxial cable (4) and an annular collar (29) for holding and locking the front end of the coaxial cable (4), said bar halves (16' and 16") being provided in the proximity of their assembly zones with radial passages in the form of slots (14, 14') coincident with passages in the outside casing (15) for the passage of the ends of the helicoidal conductor portions (2 and 2') for the purposes of the connection thereof respectively to the central wire conductor (4') and the external tubular conductor (4") of the coaxial cable (4).

18. A probe according to any one of claims 1 to 17 characterised in that the helicoidal conductor portions (2 and 2'), in particular of copper, are fitted by glueing into corresponding receiving grooves.

19. A probe according to any one of claims 1 to 16 characterised in that the helicoidal conductor portions (2 and 2') are produced by deposit of a metal layer, in particular of copper, followed by selective erosion or etching forming the helicoidal patterns constituting said conductor portions.

20. A probe according to any one of claims 1 to 8 characterised in that each antenna (1, 1') is formed together with the dielectric support structure (3) by helicoidal winding of at least one strip portion (31) of a flexible dielectric material and comprising in the mass thereof a strip (2, 2') of conductor material, said at least one strip portion (31) being applied against the inside wall of the front end (32') of a tube (32) of a flexible or semi-rigid synthetic material, under the action of the resilient thrust force resulting from a residual state of radial compression of said at least one strip portion (31) in said tube (32).

21. A probe according to claim 20 characterised in that the antenna (1) is formed by winding in opposite directions of two strip portions (31), the turns of the strip portions (31) being contiguous and radial abutments (33, 33') which are for example resilient and annular in shape being disposed at the location of the front (3') and rear (3") ends of the strip portions (31) forming the dielectric support structure (3) and the antenna (1).

22. A probe according to claim 20 characterised in that the antennae (1 and 1') are formed by winding four strip portions (31) two by two and in opposite directions, each pair of strip portions (31) comprising a longer portion and a shorter portion, the turns of the pairs of the strip portions (31) being contiguous and in contact at the location of the front (3') and rear (3") ends against radial abutments (33, 33').

23. A probe according to claim 22 characterised in that the strip portions (31) of smaller length intended to form the antenna (1') adapted for radiometric reception become progressively thinner in the direction of the free ends thereof.

24. A probe according to either one of claims 20 and 23 characterised in that the flow of thermostatic control fluid is ensured on the one hand by the tube (32) whose front end is sealingly closed and whose rear prolongation portion containing the coaxial cable (4) is connected to a thermostatic control fluid injection device (35) and on the other hand by a tubular conduit (34) disposed in the tube (32) and opening at the location of the front end of the latter, said tubular conduit (34) being connected to a device (36) for discharge or recirculation of the thermostatic control fluid.

25. A probe according to any one of claims 20 to 24 characterised in that the location of the opposite distal ends of the antenna (1) or the antennae (1, 1') it comprises supplementary pieces or inserts (40) of a dielectric material, for example in the form of rings, which are disposed in the tubular duct delimited by said at least one strip portion (31) providing the antenna (1) or the antennae (1, 1').

## Patentansprüche

1. Sonde zum Erhitzen von Gewebe durch Mikrowellen und zum Messen der Temperatur durch Mikrowellen-Radiometrie, die dazu bestimmt ist, in eine Vertiefung oder einen Kanal des menschlichen Körpers eingeführt zu werden, die die Zerstörung der umliegenden Gewebe, insbesondere der Prostatagewebe und der Harnröhrenwand, ermöglicht und im wesentlichen beinhaltet einerseits wenigstens eine langgestreckte Antenne (1, 1'), die durch wenigstens einen Leiterabschnitt (2, 2') mit schraubenartiger Ausbildung gebildet ist, der mit einer dielektrischen Trägerstruktur (3) verbunden ist und ein vorderes Ende (3') und ein hinteres Ende (3'') aufweist, und andererseits Mittel zur elektrischen Verbindung (4) für den Transfer von Mikrowellensignalen zu oder von dieser wenigstens einen Antenne (1, 1'), die in der Lage sind, mit einem entsprechenden äußeren Generator und Radiometer verbunden zu werden, und schließlich einen Katheter (5), der die Antenne(n) (1, 1') und gegebenenfalls wenigstens den der oder den Antenne(n) (1, 1') proximalen Teil der Mittel zur elektrischen Verbindung (4) bedeckt, wobei die dielektrische Trägerstruktur (3) Mittel zur Zirkulation von Fluid zur Temperierung der Antenne aufweist, wobei die Mittel zur Zirkulation mit Mitteln zur Zufuhr und Abfuhr des Fluids zur Temperierung verbunden sind, dadurch gekennzeichnet, daß die Mittel (7,7',9,10 ; 19,24 ; 20,28 ; 32,34) zur Zirkulation von Fluid zur Temperierung derart angepaßt sind, daß das Fluid auf der Höhe der Antenne oder der Antennen (1, 1') im Inneren des rohrförmigen Kanals, der von dem wenigstens einen schraubenartigen Leiterabschnitt (2,2') begrenzt ist, zirkulieren kann, indem es immer an der Seite des Abschnitts oder der Abschnitte (2, 2') liegt, die der äußeren Fläche der Sonde entgegengesetzt ist.

2. Sonde nach Anspruch 1, dadurch gekennzeichnet, daß jede Antenne (1,1') aus zwei schraubenartigen Leiterabschnitten (2 und 2') in Form von flachen, metallischen Bändern gebildet ist, die auf der Höhe ihrer nebeneinanderliegenden Enden verbunden sind und entgegengesetzte Windungssinne von den nebeneinanderliegenden Enden aufweisen, wobei die Mittel zur elektrischen Verbindung (4) in einem koaxialen Kabel bestehen.

3. Sonde nach einem der Ansprüche 1 und 2, dadurch gekennzeichnet, daß die Antenne (1) oder die Antennen (1,1') auf ihrer Außenseite mit einer Schicht oder einer Hülle, insbesondere mit Thermoretraktion, aus einem thermisch isolierenden Material, bedeckt ist.

4. Sonde nach einem der Ansprüche 1 und 2, dadurch gekennzeichnet, daß der Katheter (5) aus einem thermisch isolierenden Material gebildet ist.

5. Sonde nach einem der Ansprüche 1 bis 4, dadurch gekennzeichnet, daß der Katheter (5), auf der Höhe seines vorderen Endes, mit expandierbaren Mitteln (5') zur Positionierung der Sonde in der Vertiefung oder dem Kanal, und mit einem Mittel (5") zur Positionierung und Längsblockierung der Antenne (1) oder der Antennen (1,1') in dem Katheter (5) versehen ist.

6. Sonde nach einem der Ansprüche 1 bis 5, dadurch gekennzeichnet, daß das Fluid zur Temperierung, beim Hin- oder Rücklauf, um Mittel zur elektrischen Verbindung (4) in einer Hülle (6) zirkulieren kann, die rohrförmig ist und dadurch eine Längshülse bildet, die die Mittel zur elektrischen Verbindung (4) umgibt, wobei insbesondere das Teil von letzeren in der Vertiefung oder dem Kanal des menschlichen Körpers liegen kann.

7. Sonde nach einem der Ansprüche 1 bis 6, dadurch gekennzeichnet, daß sie zwei Antennen (1,1') aufweist, die jeweils aus zwei schraubenartigen Leiterabschnitten (2,2') gebildet sind und mit den Leitern des koaxialen Kabels (4), das Mittel zur elektrischen Verbindung bildet, verbunden sind, wobei die erste Antenne (1) auf die Emissionsfrequenz des Mikrowellengenerators eingestellt ist, und die zweite Antenne (1') auf die Empfangsfrequenz des Radiometers eingestellt ist, wobei jede der Antennen (1,1') eine bei der Einstellfrequenz der anderen der Antennen (1,1') höhere Impedanz aufweist.

8. Sonde nach Anspruch 7, dadurch gekennzeichnet, daß die Windungen der schraubenartigen Leiterabschnitte (2,2'), die die auf den radiometrischen Empfang eingestellte Antenne (1') bilden, zwischen den Windungen der schraubenartigen Leiterabschnitte (2,2'), die die auf Emission eingestellte Antenne (1) bilden, liegen, wobei die Enden der schraubenartigen Leiterabschnitte (2,2') der Antenne (1') progressiv dünner werden.

9. Sonde nach einem der Ansprüche 1 bis 8, dadurch gekennzeichnet, daß das Fluid zur Temperierung, auf der Höhe der Antenne (1) oder der Antennen (1,1'), wenigstens teilweise, auf einem Weg parallel zu und angrenzend an die schraubenartigen Leiterabschnitte (2 und 2') zirkulieren kann.

10. Sonde nach Anspruch 9, dadurch gekennzeichnet, daß die dielektrische Trägerstruktur (3) in einem langgestreckten Träger mit zylindrischer Form besteht und auf seiner äußeren Fläche eine Rille (7) mit schraubenartiger Ausbildung aufweist, die auf ihren beiden Seitenflächen, in der Nähe der äußeren Fläche des Trägers, mit gegenüberliegenden, fortlaufenden Schultern (8) versehen ist, wobei die Rille (7) einerseits, auf der Höhe ihres Endes, das dem hinteren Ende (3") des Trägers nahe ist, mit einer Leitung (9) zur Speisung mit Fluid zur Temperierung, und andererseits, auf der Höhe ihres Endes, das dem vorderen Ende (3') des Trägers (3) nahe ist, mit einer Leitung (10) zum Ablassen des Fluids zur Temperierung verbunden ist, die längs in dem Träger (3) ausgespart ist und in einer die Mittel zur elektrischen Verbindung (4) umgebenden, rohrförmigen Hülle(6) endet.

11. Sonde nach Anspruch 10, dadurch gekennzeichnet, daß die dielektrische Trägerstruktur (3) gebildet ist durch zwei Trägerhälften (11,11'), die koaxial durch teilweise Ineinanderschieben und Kleben auf der Ebene des mittleren Bereichs der Antenne (1) oder der Antennen (1,1') zusammengefügt sind, nämlich einerseits eine erste Trägerhälfte (11), die den oder die ersten schraubenartigen Leiterabschnitt(e) (2) trägt und eine axiale Längsperforation (12) für die Aufnahme des vorderen Abschnitts des koaxialen Kabels (4), das die Mittel zur elektrischen Verbindung bildet, aufweist, sowie einen ringförmigen Flansch (13) zum Halten und Blockieren des vorderen Endes des Abschnitts des koaxialen Kabels (4), und andererseits eine zweite Trägerhälfte (11'), die den oder die zweite(n) schraubenartigen Leiterabschnitt(e) (2') trägt, wobei die Trägerhälften (11,11'), in der Nähe ihrer Verbindungsbereiche, mit radialen Durchgängen (14,14') in Form von Schlitzen für den Durchgang der Enden der schraubenartigen Leiterabschnitte (2,2') zu ihrer Verbindung, jeweils mit dem zentralen, drahtförmigen Leiter (4') bzw. dem äußeren rohrförmigen Leiter (4") des koaxialen Kabels (4), versehen sind.

12. Sonde nach einem der Ansprüche 1 bis 8, dadurch gekennzeichnet, daß der langgestreckte dielektrische Träger (3) durch eine auf einem inneren Dorn (16) befestigte, äußere Hülse (15) gebildet ist, wobei der äußere Dorn (15) die schraubenartigen Leiterabschnitte (2 und 2') trägt und der innere Dorn (16), der in seinem hinteren Teil eine langgestreckte axiale Perforation (17) aufweist, den vorderen Abschnitt des koaxialen Kabels (4), das die Mittel zur elektrischen Verbindung bildet und mit einer das koaxiale Kabel (4) umgebenden, rohrförmigen Hülle (6) verbunden ist, aufnimmt.

13. Sonde nach Anspruch 12, dadurch gekennzeichnet, daß der innere Dorn (16) in der äußeren Hülse (15) befestigt ist mit Vorsehen einer longitudinalen, ringförmigen Lücke (19) oder länglichen Abschnitten (20) mit einer ringförmigen Lücke zwischen diesen beiden Hülsen (15 und 16), wobei das Fluid zur Temperierung in dieser Lücke (19) oder diesen Lückenabschnitten (20) zirkuliert.

14. Sonde nach Anspruch 13, dadurch gekennzeichnet, daß der innere Dorn (16) einerseits, auf seiner Außenseite, und wenigstens auf der Höhe seiner Längsenden, versehen ist mit vorragenden radialen Abschnitten (21), die in der Länge begrenzt sind, mit einem regelmäßigen Abstand um die Enden angeordnet sind und auf die Innenseite der äußeren Hülse (15) nach Verbinden durch Ineinanderschieben des Dorns (16) in die Hülse (15) drücken, und andererseits versehen ist mit vorragenden frontalen Abschnitten (22) auf der Höhe des vorderen Endes des Dorns (16), die nach Verbinden, auf eine Wand zum Verschließen (23) des vorderen Endes der äußeren Hülse (15) drücken und Durchgänge aussparen zur Verbindung (22') zwischen der ringförmigen Lücke (19), die zwischen dem Dorn (16) und der Hülse (15) existiert, und einer Leitung (24) zum Ablassen des Fluids zur Temperierung, die sich längs in dem inneren Dorn (16) erstreckt, die auf der vorderen Frontseite des Dorns (16) endet und mit der den vorderen Abschnitt des koaxialen Kabels (4) aufnehmenden, axialen Perforation (17) verbunden ist.

15. Sonde nach Anspruch 13, dadurch gekennzeichnet, daß der innere Dorn (16) auf seiner Außenseite mit vorragenden Abschnitten (25) versehen ist, die auf der gesamten Länge des Dorns (16) verlaufen und untereinander Abschnitte mit einer ringförmigen Lücke (20) begrenzen, die getrennte Kanäle zur Zirkulation von Fluid zur Temperierung mit Ringquerschnitt im Abschnitt bilden, wobei einige der vorragenden Abschnitte (25) im Abstand zu einer vorderen Wand (26) zur Begrenzung und frontalem Verschluß der Kanäle enden, derart, daß für jeden Kanal ein Durchgang (27) zwischen dem jeweiligen Kanal und wenigstens einem benachbarten Kanal, auf Höhe des vorderen Endes der inneren Hülse (16)ausgespart ist.

16. Sonde nach Anspruch 15, dadurch gekennzeichnet, daß einige der Abschnitte mit ringförmiger Lücke (20), in der Nähe ihres hinteren Endes, mit der axialen Perforation (17), die den vorderen Abschnitt des koaxialen Kabels (4) mittels radialer Öffnungen (28), insbesondere in Form von Längslöchern oder Schlitzen aufnimmt, in Verbindung stehen.

17. Sonde nach einem der Ansprüche 13 bis 16, dadurch gekennzeichnet, daß der innere Dorn (16) aus zwei Dornhälften (16' und 16") gebildet ist, die koaxial durch teilweise Ineinanderschieben und Kleben auf Höhe des mittleren Bereichs des Trägers (3) verbunden sind, wobei die den hinteren Teil des Dorns (16) bildende Hälfte (16") die axiale Perforation (17) zur Aufnahme des vorderen Abschnitts des koaxialen Kabels (4) und einen ringförmigen Flansch (29) zum Halten und Blockieren des vorderen Endes des koaxialen Kabels (4) aufweist, wobei die Dornhälften (16 und 16'), in der Nähe ihrer Verbindungsbereiche, mit radialen Durchgängen in Form von Schlitzen (14, 14') versehen sind, die übereinstimmen mit Durchgängen der äußeren Hülse (15), für den Durchgang der Enden der schraubenartigen Leiterabschnitte (2 und 2') zu ihrer Verbindung, jeweils mit dem zentralen, drahtförmigen Leiter (4') bzw. dem äußeren rohrförmigen Leiter (4") des koaxialen Kabels (4).

18. Sonde nach einem der Ansprüche 1 bis 17, dadurch gekennzeichnet, daß die schraubenartigen Leiterabschnitte (2 und 2'), insbesondere aus Kupfer, durch Kleben in entsprechenden Aufnahmerillen eingefügt sind.

19. Sonde nach einem der Ansprüche 1 bis 16, dadurch gekennzeichnet, daß die schraubenartigen Leiterabschnitte (2 und 2') hergestellt werden durch Ablagerung einer Metallschicht, insbesondere Kupfer, gefolgt von einer Ätzung oder einer selektiven Abtragung, wodurch die die Leiterabschnitte bildenden schraubenartigen Erscheinungen gebildet werden.

20. Sonde nach einem der Ansprüche 1 bis 8, dadurch gekennzeichnet, daß jede Antenne (1,1'), zusammen mit der dielektrischen Trägerstruktur (3), durch schraubenartiges Wikkeln wenigstens eines Streifenabschnitts (31) aus einem biegsamen dielektrischen Material gebildet ist und in ihrer Masse einen Streifen Leitermaterial (2,2') aufweist, wobei der wenigstens eine Abschnitt des Streifen (31) gegen die Innenwand des vorderen Endes (32') eines Rohrs (32) aus einem weichen oder halbsteifen synthetischen Material gedrückt wird, dies unter Einwirkung des elastischen Drucks, der aus einem Zustand der restlichen radialen Kompression des wenigstens einen Streifenabschnitts (31) in dem Rohr (32) resultiert.

21. Sonde nach Anspruch 20, dadurch gekennzeichnet, daß die Antenne (1) durch Wickeln, in entgegengesetzten Richtungen, von zwei Streifenabschnitten (31) gebildet ist, wobei die Windungen der Streifenabschnitte (31) aneinanderstoßen und radiale Anschläge (33, 33'), beispielsweise elastische und ringförmige, auf der Höhe des vorderen (3) und hinteren (3") Endes der Streifenabschnitte (31), die die dielektrische Trägerstruktur (3) und die Antenne (1) bilden, angeordnet sind.

22. Sonde nach Anspruch 20, dadurch gekennzeichnet, daß die Antennen (1 und 1') durch Wickeln, zwei und zwei und in entgegensetzten Richtungen, aus vier Streifenabschnitten (31) gebildet sind, wobei jedes Paar von Streifenabschnitten (31) eine längeren Abschnitt und einen kürzeren Abschnitt aufweist, wobei die Windungen der Paare der Streifenabschnitte (31) aneinanderstoßen und auf der Höhe des vorderen (3') und hinteren (3") Endes gegen die radialen Anschläge (33, 33') drücken.

23. Sonde nach Anspruch 22, dadurch gekennzeichnet, daß die Streifenabschnitte (31) mit geringerer Länge, die dazu bestimmt sind, die für den radiometrischen Empfang eingestellte Antenne zu bilden, in Richtung ihrer freien Enden progressive dünner werden.

24. Sonde nach einem der Ansprüche 20 oder 23, dadurch gekennzeichnet, daß die Zirkulation des Fluids zur Temperierung gewährleistet ist einerseits durch das Rohr (32), dessen vorderes Ende dicht verschlossen ist und dessen hintere Verlängerung, die das koaxiale Kabel (4) enthält, mit einer Vorrichtung (35) zum Einführen des Fluids zur Temperierung verbunden ist, und andererseits durch eine rohrförmige Leitung (34), die in dem Rohr (32) liegt und auf der Höhe des vorderen Endes von letzterem endet, wobei die rohrförmige Leitung (34) mit einer Vorrichtung (36) zum Ablassen oder Rezirkulieren des Fluids zur Temperierung verbunden ist.

25. Sonde nach einem der Ansprüche 20 bis 24, dadurch gekennzeichnet, daß sie, auf Höhe der distalen Enden, die der Antenne (1) oder den Antennen (1,1') entgegengesetzt sind, zusätzliche Teile oder Einsätze (40) aus einem dielektrischen Material aufweist, beispielsweise in Form von Ringen, die in dem rohrförmigen Kanal angeordnet ist/sind, der von dem wenigstens einen die Antenne (1) oder die Antennen (1,1') bildenden Streifenabschnitt (31) begrenzt wird.
